# EUROPEAN PATENT APPLICATION

(11) **EP 4 209 593 A1**
(43) Date of publication of application: **12.07.2023**
(21) Application number: 21864470.6
(22) Date of filing: 06.09.2021
(51) Int. Cl.: C12N 15/12, C12N 5/0735, C12N 5/10, C12N 15/62, C12N 15/86

(54) **QUALITY IMPROVING AGENT FOR IPS CELLS, METHOD OF PRODUCING IPS CELLS, IPS CELLS, AND COMPOSITION FOR PRODUCING IPS CELLS**

(30) Priority: 04.09.2020 JP 2020149447
(71) Applicant: Heartseed Inc., Tokyo 160-0015 (JP); Keio University, Tokyo, 108-8345 (JP); Kyoto University, Kyoto-shi, Kyoto 606-8501 (JP); ID Pharma Co., Ltd., Tokyo 102-0071 (JP)
(72) Inventor: KUNITOMI Akira, Kyoto-shi, Kyoto 606-8501 (JP); FUKUDA Keiichi, Tokyo 160-8582 (JP); YUASA Shinsuke, Tokyo 160-8582 (JP); SHU Tsugumine, Tokyo 102-0071 (JP)
(74) Representative: Script IP Limited
(86) International application number: PCT/JP2021/032734
(87) International publication number: WO 2022/050419

(57) **Abstract**

There is provided a quality improving agent for iPS cells, including a polynucleotide, in which the polynucleotide contains an H1foo gene and a regulatory sequence that is capable of regulating at least one of the amount and the period of existence of an H1foo protein expressed from the H1foo gene in cells when the H1foo gene is transduced into the cells.

## Description

### [Technical Field]

The present invention relates to a quality improving agent for iPS cells, a method of producing iPS cells, iPS cells, and a composition for producing iPS cells.

Priority is claimed on Japanese Patent Application No. 2020-149447, filed September 4, 2020, the content of which is incorporated herein by reference.

### [Background Art]

iPS cells (induced pluripotent stem cells; also called "induced pluripotent stem cell" or "induced pluripotent stem cells") are produced from a somatic cell by introducing Oct3/4, Sox2, Klf4, and c-Myc (Non-Patent Document 1, Patent Document 1). This can be achieved by reprogramming the transcriptional network and epigenetic signature of parental somatic cells. iPS cells offer a variety of benefits for basic research, pharmaceutical innovation, and regenerative medicine. However, it is still a serious problem that a cell population of generated iPS cells has heterogeneous quality as compared with a cell population of embryonic stem cells (ES cells). For example, in a case of ES cells, there is a small variation in the properties between the cells, and almost any cell can be differentiated into target cells. On the other hand, iPS cells vary greatly in properties between the cells, and thus cells that cannot be differentiated into target cells often appear. For basic research and clinical purposes, it is important to obtain a cell population of iPS cells that vary little between the cells, where the iPS cells exhibit high quality as well.

Many attempts have been made to solve the problem of the heterogeneous quality of a cell population of iPS cells. For example, Patent Document 2 states that in a case of introducing predetermined amounts of an Oct3/4 gene, a Klf4 gene, a c-Myc gene, and a Sox2 gene into somatic cells a predetermined number of times, it is possible to improve the production efficiency and stability of iPS cells. Patent Document 3 states that in a case of introducing, into somatic cells, a Prdm 14 gene or a gene product thereof, an Esrrb gene or a gene product thereof, and a Sall4a gene or a gene product thereof, in addition to the Oct3/4 gene or a gene product thereof, the Sox2 gene or a gene product thereof, the Klf4 gene or a gene product thereof, and the c-Myc gene or a gene product thereof, it is possible to efficiently produce iPS cells with excellent quality in a short period of time. Patent Document 4 states that in a case of introducing, into somatic cells, a Jarid2 mutant gene or a gene product thereof in addition to the Oct3/4 gene or a gene product thereof, the Sox2 gene or a gene product thereof, the Klf4 gene or a gene product thereof, and the c-Myc gene or a gene product thereof, it is possible to efficiently produce iPS cells with excellent quality in a short period of time. However, the quality of iPS cells still has room for improvement. Under such circumstances, the development of a method of producing iPS cells having a higher quality and smaller variation in quality has been demanded.

A linker histone H1 family binds to a linker DNA and gives rise to a higher order chromatin structure in order to regulate gene expression. Members of the linker histone H1 family include histones H1a, H1b, H1c, H1d, H1e, Hlfoo, H1x, H1.0, H1t, H1T2, and HILS1. Most members of the linker histone family consist of somatic cell linker histones that cause condensation of chromatin. As a result, such a structure generally suppresses global gene transcription activity (Non-Patent Documents 2 and 3). The inventors of the present invention have found that in a case of inducing iPS cells from somatic cells, it is possible to produce iPS cells having high quality and a small variation in quality by introducing the H1foo gene, which is a member of the linker histone H1 family, in addition to the above genes (Patent Document 5).

### [Citation List]

### [Patent Documents]

[Patent Document 1]
   Japanese Patent No. 4183742
[Patent Document 2]
   Japanese Unexamined Patent Application, First Publication No. 2011-004674
[Patent Document 3]
   Japanese Unexamined Patent Application, First Publication No. 2014-217344
[Patent Document 4]
   Japanese Unexamined Patent Application, First Publication No. 2014-217345
[Patent Document 5]
   PCT International Publication No. WO2017/010080

### [Non-Patent Documents]

[Non-Patent Document 1]
   Takahashi, K. & Yamanaka, S. Induction of pluripotent stem cells from mouse embryonic and adult fibroblast cultures by defined factors. Cell 126, 663-676 (2006)
[Non-Patent Document 2]
   Steinbach, O.C., Wolffe, A.P. & Rupp, R.A. Somatic linker histones cause loss of mesodermal competence in Xenopus. Nature 389, 395-399 (1997)
[Non-Patent Document 3]
   Hebbar, P.B. & Archer, T.K. Altered histone H1 stoichiometry and an absence of nucleosome positioning on transfected DNA. The Journal of biological chemistry 283, 4595-4601 (2008)

### [Summary of Invention]

### [Technical Problem]

According to the method of producing iPS cells described in Patent Document 5, it is possible to produce iPS cells having high quality and a small variation in quality as compared with the methods in the related art. However, the number of colonies obtained after iPS cell induction is on the same level as the methods in the related art.

An object of the present invention is to provide a quality improving agent for iPS cells, a method of producing iPS cells, iPS cells produced by such a production method, and a composition for producing iPS cells, which make it possible to increase the number of colonies obtained after iPS cell induction.

### [Solution to Problem]

As a result of repeating intensive studies to solve the above problems, the inventors of the present invention found that in a case of regulating any one of period of existence or amount of an H1foo protein in cells, the H1foo protein being expressed from the H1foo gene introduced into somatic cells, the number of colonies after iPS cell induction increases significantly, thereby completing the present invention.

The present invention includes the following aspects.
[1] A quality improving agent for iPS cells, including: one or more polynucleotides, wherein the one or more polynucleotides contain an H1foo gene and a regulatory sequence that is capable of regulating in at least one of the amount and the period of existence of an H1foo protein expressed from the H1foo gene in cells when the H1foo gene is transduced into the cells.
[2] The quality improving agent for iPS cells according to [1], wherein the polynucleotide is inserted into an expression vector in a state capable of expressing the H1foo gene in cells when the polynucleotide is transduced into the cells.
[3] The quality improving agent for iPS cells according to [2], wherein the expression vector is a Sendai virus vector.
[4] The quality improving agent for iPS cells according to any one of [1] to [3], wherein the regulatory sequence includes a nucleotide sequence encoding a destabilization domain, the destabilization domain is a domain that promotes proteasomal degradation of a fusion protein containing the destabilization domain, and the regulatory sequence is linked to the H1foo gene such that the fusion protein of the destabilization domain and the H1foo protein is capable of being expressed.
[5] The quality improving agent for iPS cells according to any one of [1] to [4], wherein the regulatory sequence includes a promoter sequence that regulates the transcription of the H1foo gene in response to a chemical stimulus.
[6] A quality improving agent for iPS cells, including: a fusion protein of an H1foo protein and a destabilization domain, wherein the destabilization domain is a domain that promotes proteasomal degradation of the fusion protein.
[7] A method of producing iPS cells including: a step of introducing into somatic cells, a nuclear reprogramming factor and the quality improving agent for iPS cells according to any one of [1] to [6].
[8] The method of producing iPS cells according to [7], wherein the iPS cells are prime-type or naive-type iPS cells.
[9] The method of producing iPS cells according to [7] or [8], wherein the nuclear reprogramming factor includes at least one selected from the group consisting of a gene of an Oct gene family, a gene of a Sox gene family, a gene of a Klf gene family, a gene of a Myc gene family, a gene of a Lin gene family, a Nanog gene, and a gene product thereof.
[10] The method of producing iPS cells according to any one of [7] to [9], wherein the nuclear reprogramming factor consists of an Oct3/4 gene, a Sox2 gene, a Klf4 gene, L-Myc or c-Myc, and a gene product thereof.
[11] The method of producing iPS cells according to any one of [7] to [9], wherein the nuclear reprogramming factor includes at least one gene selected from the group consisting of a gene of an Oct gene family, a gene of a Sox gene family, a gene of a Klf gene family, a gene of a Myc gene family, a gene of a Lin gene family, and a Nanog gene, and the at least one gene is inserted into an expression vector in a state capable of expressing in cells when the at least one gene is transduced into the cells
[12] The method of producing iPS cells according to [11], wherein the expression vector is a Sendai virus vector.
[13] A method of producing iPS cells, including: a step of introducing a nuclear reprogramming factor and an H1foo gene into somatic cells, wherein an H1foo protein expressed from the H1foo gene transduced into somatic cells is regulated in at least one of the amount and the period of existence of that in the somatic cells.
[14] The method of producing iPS cells according to [13], wherein the H1foo gene is inserted into an expression vector in a state capable of expressing the H1foo gene in cells when the H1foo gene is transduced into the cells.
[15] The method of producing iPS cells according to [13] or [14], wherein the nuclear reprogramming factor is at least one gene selected from the group consisting of a gene of an Oct gene family, a gene of a Sox gene family, a gene of a Klf gene family, a gene of a Myc gene family, a gene of a Lin gene family, and a Nanog gene, and the at least one gene is encoded in an expression vector that is capable of expressing the at least one gene.
[16] The method of producing iPS cells according to [14] or [15], wherein the expression vector is a Sendai virus vector.
[17] iPS cells that are produced by the method of producing iPS cells according to any one of [7] to [16].
[18] A composition for producing iPS cells, including: a nuclear reprogramming factor; and the quality improving agent for iPS cells according to any one of [1] to [6].
[19] A method of producing iPS cells, including: a step of introducing a nuclear reprogramming factor and a substance inducing the reprogramming of somatic cells either from 2 to 15 days after being introduced into the somatic cells together with the reprogramming factor, and suppressing natural immunity, into the somatic cells.

### [Advantageous Effects of Invention]

According to the present invention, a quality improving agent for iPS cells, a method of producing iPS cells, iPS cells produced by such a production method, and a composition for producing iPS cells are provided. According to the present invention, it is possible to increase the number of colonies obtained after iPS cell induction, and
a large number of cells having high quality as iPS cells, such as high pluripotency, are contained in an iPS cell population produced in such a manner.

### [Brief Description of Drawings]

FIG. 1 shows structures of three Sendai virus vectors (SeV18+HlFOO/TS15AF, SeV18+H1FOO-DD/TS15ΔF, and SeV18+DD-H1FOO/TS15ΔF) containing an H1FOO gene used in Examples.
FIG. 2 shows results of comparing expression levels of H1FOO (2), an H1FOO-DD fusion protein (3), and a DD-H1FOO fusion protein (4) by Western blotting, which are obtained by introducing any of the three Sendai virus vectors shown in FIG. 1 into human skin fibroblasts. In "(1) Control" of FIG. 2, a Sendai virus vector containing an Azami-Green gene is used instead of the H1FOO gene in "(a) SeV18+H1FOO/TS15ΔF" shown in FIG. 1 (the same applies hereinafter).
FIG. 3 shows the number of alkaline phosphatase (ALP)-positive colonies in prime-type iPS cells prepared from human skin fibroblasts. The prime-type iPS cells have been prepared using any Sendai virus vector of (a) to (c) shown in FIG. 1 together with Sendai virus vectors containing an Oct3/4 gene, a Sox2 gene, a Klf4 gene, and an L-Myc gene.
FIG. 4A shows the number of ALP-positive colonies in prime-type iPS cells prepared from human peripheral blood mononuclear cells. The prime-type iPS cells have been prepared using SeV18+H1FOO-DD together with Sendai virus vectors containing an Oct3/4 gene, a Sox2 gene, a Klf4 gene, and an L-Myc gene.
FIG. 4B shows the number of ALP-positive colonies in naive-type iPS cells prepared from human skin fibroblasts. The naive-type iPS cells have been prepared using SeV18+H1FOO-DD together with Sendai virus vectors containing an Oct3/4 gene, a Sox2 gene, a Klf4 gene, and an L-Myc gene.
FIG. 4C shows the number of ALP-positive colonies in naive-type iPS cells prepared from human peripheral blood mononuclear cells. The naive-type iPS cells have been prepared using SeV18+H1FOO-DD together with Sendai virus vectors containing an Oct3/4 gene, a Sox2 gene, a Klf4 gene, and an L-Myc gene.
FIG. 5A shows results of comparatively evaluating the variation in gene expression levels between (1) Control-iPS and (3) H1FOO-DD-iPS in the prime-type iPS cells prepared from human skin fibroblasts.
FIG. 5B shows results of comparatively evaluating the variation in gene expression levels between (1) Control-iPS and (3) H1FOO-DD-iPS in the naive-type iPS cells prepared from human skin fibroblasts.
FIG. 6A shows results of comparatively evaluating the variation in DNA methylation between (1) Control-iPS and (3) H1FOO-DD-iPS in the prime-type iPS cells prepared from human skin fibroblasts.
FIG. 6B shows results of comparatively evaluating the variation in DNA methylation between (1) Control-iPS and (3) H1FOO-DD-iPS in the naive-type iPS cells prepared from human skin fibroblasts.
FIG. 7A shows measurement examples obtained by measuring the proportion of TNNT2-positive cells by flow cytometry in cells induced to differentiate from the prime-type iPS cells using a myocardial differentiation induction medium. The left figure is a measurement example of (1) Control-iPS, and the right figure is a measurement example of (3) H1FOO-DD-iPS.
FIG. 7B shows results of comparatively evaluating the proportion of TNNT2-positive cells between (1) Control-iPS and (3) H1FOO-DD-iPS in cells induced to differentiate from the prime-type iPS cells using a myocardial differentiation induction medium.
FIG. 8 shows results of semi-exhaustively evaluating, by qPCR, the expression of endoderm-related markers between (1) Control-iPS and (3) H1FOO-DD-iPS in cells induced to differentiate into the endoderm from the prime-type iPS cells.
FIG. 9 shows results of comparatively evaluating, by qPCR, the expression of ASGR1 between (1) Control-iPS and (3) H1FOO-DD-iPS in cells induced to differentiate into hepatocytes from the prime-type iPS cells.
FIG. 10 shows comparative results of evaluating, by ELISA, the secretion of Albumin between (1) Control-iPS and (3) H1FOO-DD-iPS in cells induced to differentiate into hepatocytes from the prime-type iPS cells.
FIG. 11A shows measurement examples obtained by measuring the proportion of cells positive for both PDGFRA and ANPEP by flow cytometry in cells induced to differentiate from the naive-type iPS cells using a primitive endoderm differentiation induction medium. The left figure is a measurement example of (1) Control-iPS, and the right figure is a measurement example of (3) H1FOO-DD-iPS.
FIG. 11B shows results of comparatively evaluating the proportion of cells positive for both PDGFRA and ANPEP between (1) Control-iPS and (3) H1FOO-DD-iPS in cells induced to differentiate from the naive-type iPS cells using a primitive endoderm differentiation induction medium.
FIG. 12A shows results of comparatively evaluating the spare respiratory capacity between (1) Control-iPS and (3) H1FOO-DD-iPS in the naive-type iPS cells.
FIG. 12B shows results of comparatively evaluating the oxygen consumption rate (OCR) and the extracellular acidification rate (ECAR) between (1) Control-iPS and (3) H1FOO-DD-iPS in the naive-type iPS cells.
FIG. 13A shows examples of fluorescence microscopic images of mRNA-FISH in the naive-type iPS cells, obtained by using UTX, HUWE1, and XIST probes. The left figure is an example of HUWEI+/+XIST+/+. The right figure is an example of HUWEI+/-XIST-/-.
FIG. 13B shows results of comparatively evaluating the expression patterns of HUWE1 and XIST between (1) Control-iPS and (3) H1FOO-DD-iPS in naive-type iPS cells.
FIG. 14 shows results of measuring the expression level of FKBP1A by qRT-PCR.

HDF: human skin fibroblast (TIG) 120; H9 ESC: H9 ES cell; H1FOO OE HDF: cells on 2nd day after introduction of H1FOO-DD into human skin fibroblasts; OSKL day 2: cells on 2nd day after introduction of nuclear reprogramming factors (OCT4/SOX2/KLF4/LMYC) into human skin fibroblasts; OSKLH day 2: cells on 2nd day after introduction of nuclear reprogramming factors (OCT4/SOX2/KLF4/LMYC) and H1FOO-DD into human skin fibroblasts.

### [Description of Embodiments]

The term "comprise" means that it may contain components other than the subject component t. The term "consist of" means not including components other than the subject component The term "consist essentially of" does not include a constitutional element other than the constitutional element to be targeted, as an aspect in which the constitutional element exhibits a special function (such as an aspect in which the effect of the invention is lost). In the present specification, in a case where a description is made using "comprise", an aspect of "consist of" and an aspect of "essentially consist of" are encompassed.

A protein (a polypeptide), a peptide, a polynucleotide (DNA or RNA), a vector, and a cell can be those that have been isolated. The term "isolated" means a state of being separated from the natural state. A protein (a polypeptide), a peptide, a polynucleotide (DNA or RNA), a vector, and a cell, which are described in the present specification, can be an isolated protein (an isolated polypeptide), an isolated peptide, an isolated polynucleotide (an isolated DNA or an isolated RNA), an isolated vector, and an isolated cell.

The term "gene" means a polynucleotide containing at least one open reading frame encoding a specific protein. A gene may contain exons only, or it may contain exons and any one or more of introns, 5'UTR, and 3'UTR.

The term "functionally linked" that is used for a polynucleotide means a state where a first base sequence is positioned sufficiently close to a second base sequence and thus the first base sequence can influence on a region which is under the regulation of the second base sequence or the second base sequence. For example, the description that a polynucleotide is "functionally linked to a promoter" means that a polynucleotide is linked to be expressed under the regulation of the promoter. In a case where a promoter is positioned upstream of a gene, the gene is generally linked functionally to the promoter.

The phrase "in a state capable of expressing" means that a polynucleotide is in a state where it is capable of being transcribed in a cell into which the polynucleotide has been introduced.

The term "expression vector" refers to a vector containing a subject polynucleotide, which includes a system for making the subject polynucleotide be in a state capable of being expressed in a cell into which the vector has been introduced.

### [Quality improving agent for iPS cells]

### <First embodiment>

In one embodiment, the present invention provides a quality improving agent for iPS cells. The quality improving agent for iPS cells according to the present embodiment contains a polynucleotide having an H1foo gene and a nucleotide sequence (hereinafter, referred to as a "regulatory sequence") that is capable of regulating at least one of the amount and the period of the existence of the H1foo protein expressed from the H1foo gene in cells when the H1foo gene is transduced into the cells.

In a case of being introduced into somatic cells together with a nuclear reprogramming factor described later, the quality improving agent for iPS cells according to the present embodiment can improve the quality of iPS cells induced from the somatic cells. Examples of the "quality of iPS cells" include various properties such as that the expression of undifferentiation markers (Nanog, Tra-1-60, ALP, and the like) in iPS cells is high, that embryoid body forming ability is high, that the uniformity of sizes of embryoid bodies formed from iPS cells is high and aberrant methylation is rare, that the chimera forming ability in mouse iPS cells is high, and the ability to differentiate into differentiated cells (for example, cardiomyocytes) is high; and the uniformity of such various properties as described above, among iPS cells. In a case of being introduced into somatic cells together with a nuclear reprogramming factor, the quality improving agent for iPS cells according to the present embodiment exhibits an effect such as improvement of various properties of iPS cells as described above or improvement of uniformity of various properties among iPS cells, as compared with a case where only the nuclear reprogramming factor is introduced. In particular, the quality improving agent for iPS cells according to the present embodiment exhibits an effect such as an improvement of the ability to generate prime-type iPS cells that express ALP or an improvement of the ability to generate naive-type iPS cells that express ALP. In addition, in a case of being introduced into somatic cells together with a nuclear reprogramming factor, the quality improving agent for iPS cells according to the present embodiment exhibits effects in the generated iPS cells, such as improvement of uniformity of gene expression, improvement of uniformity of DNA methylation, improvement of the ability to differentiate into target cells, and improvement of uniformity of such properties, as well as improvement of the ability to differentiate into a differentiated cell population having high uniformity and improvement of expression of a naive-type phenotype (metabolic function, HUWE1/XIST expression pattern, or the like). The uniformity in differentiated cells can be checked, for example, by examining the variation in expression levels of differentiated cell markers. Differentiated cells are not particularly limited; however, examples thereof include an endodermal cell, a mesodermal cell, an ectodermal cell, a cardiomyocyte, a hepatocyte, a renal cell, a muscle cell, a fibroblast, a nerve cell, an immune cell (a lymphocyte or the like), a vascular cell, an ocular cells (a retinal pigment epithelial cell or the like), a blood cell (a megakaryocyte, an erythrocyte, or the like), other various tissue cells, and progenitor cells thereof.

### (H1foo gene)

In the present specification, the term "H1foo gene" means a polynucleotide that encodes an H1foo protein. The biological species from which the H1foo gene is derived is not particularly limited and can be appropriately selected depending on the intended purpose. Examples thereof include any mammals such as a human, a mouse, a rat, a cow, a sheep, a horse, and a monkey. The sequence information of the H1foo gene can be obtained from known databases. For example, it is available from GenBank as accession number BC047943 (human) or BC137916 (mouse). A nucleotide sequence of the human H1foo gene with the above-described accession number is set forth in SEQ ID NO: 1, and an amino acid sequence of the human H1foo protein encoded by the nucleotide sequence is set forth in SEQ ID NO: 2. A nucleotide sequence of the mouse H1foo gene with the above-described accession number is set forth in SEQ ID NO: 3, and an amino acid sequence of the mouse H1foo protein is set forth in SEQ ID NO: 4. In the present specification, in a case where a description is made in all capital letters as "H1FOO", it refers to the human H1foo gene or H1foo protein. In a case where a description is made as "Hlfoo", it includes H1foo genes or H1foo proteins of all biological species including a human.

The H1foo gene is not limited to a wild-type H1foo gene and may include those having a mutation (any one of deletion, substitution, insertion, and addition, or a combination thereof). The number of mutations is not particularly limited as long as a nucleotide sequence that encodes a protein having the H1foo activity is used. In the present specification, the term "H1foo activity" refers to at least one of functions of the wild-type H1foo protein. Examples of the H1foo activity include the activity of binding to a linker DNA that connects nucleosomes. The H1foo activity is more preferably an activity that binds to a linker DNA connecting nucleosomes and maintains a region of the linker DNA to be in a relaxed state.

Examples of the H1foo gene include the following (a) to (g).
(a) A wild-type H1foo gene (for example, a polynucleotide consisting of a nucleotide sequence set forth in SEQ ID NO: 1 or 3)
(b) A polynucleotide that consists of a nucleotide sequence encoding a wild-type H1foo protein (for example, a protein consisting of an amino acid sequence set forth in SEQ ID NO: 2 or 4)
(c) A polynucleotide that encodes a protein consisting of an amino acid sequence, in which one or a plurality of amino acids are mutated in an amino acid sequence of a wild-type H1foo protein (for example, an amino acid sequence set forth in SEQ ID NO: 2 or 4), and having the H1foo activity
(d) A polynucleotide that encodes a protein consisting of an amino acid sequence, which has 70% or more of sequence identity with an amino acid sequence of a wild-type H1foo protein (for example, an amino acid sequence set forth in SEQ ID NO: 2 or 4), and having the H1foo activity
(e) A polynucleotide that encodes a protein consisting of a nucleotide sequence, in which one or a plurality of nucleotides are mutated in a nucleotide sequence of a wild-type H1foo gene (for example, a nucleotide sequence set forth in SEQ ID NO: 1 or SEQ ID NO: 3), and having H1foo activity
(f) A polynucleotide that consists of a nucleotide sequence having 70% or more of sequence identity with a nucleotide sequence of a wild-type H1foo gene (for example, a nucleotide sequence represented by SEQ ID NO: 1 or SEQ ID NO: 3) and encodes a protein having H1foo activity
(g) A polynucleotide that hybridizes with a wild-type H1foo gene (for example, a polynucleotide containing a nucleotide sequence set forth in SEQ ID NO: 1 or SEQ ID NO: 3) under stringent conditions and encodes a protein having H1foo activity

In (c) and (e) described above, "mutation" may be any one of deletion, substitution, addition, and insertion or may be a combination thereof.

In (c) described above, "a plurality of amino acids" is not particularly limited as long as the resultant protein has the H1foo activity. However, examples thereof include 2 to 30 amino acids, where 2 to 20 amino acids are preferable, 2 to 10 amino acids are more preferable, 2 to 5 amino acids are still more preferable, and 2 or 3 amino acids are particularly preferable.

In (e) described above, "a plurality of nucleotides" is not particularly limited as long as the resultant polynucleotide encodes a protein having the H1foo activity. However, examples thereof include 2 to 60 nucleotides, where 2 to 50 nucleotides are preferable, 2 to 40 nucleotides or 2 to 30 nucleotides are more preferable, 2 to 20 nucleotides or 2 to 10 nucleotides are still more preferable, and 2 to 5 nucleotides or 2 or 3 nucleotides are particularly preferable.

In (d) and (f) described above, the sequence identity is not particularly limited as long as it is 70% or more. However, it is preferably 80% or more, more preferably 85% or more, and still more preferably 90% or more, 95% or more, 96% or more, 97% or more, 98% or more, or 99% or more. The sequence identity between amino acid sequences or between nucleotide sequences is determined by juxtaposing two amino acid sequences or two nucleotide sequences so that the corresponding amino acids or nucleotides are matched most while inserting a gap in the portion corresponding to an insertion and a deletion and obtaining a proportion of the matched amino acids or nucleotides to the entire amino acid sequence or entire nucleotide sequence from which gaps of the obtained alignment have been excluded. The sequence identity between amino acid sequences or between nucleotide sequences can be determined using various homology search software known in the related technical field. For example, a sequence identity value for amino acid sequences or nucleotide sequences can be obtained by a calculation based on the alignment obtained by the known homology search software BLASTP or BLASTN.

In (g) described above, examples of the terms "stringent conditions" include the conditions described in Molecular Cloning-A LABORATORY MANUAL THIRD EDITION (Sambrook et al., Cold Spring Harbor Laboratory Press). Examples thereof include conditions under which hybridization is carried out by performing incubation at 42°C to 70°C for several hours to overnight in a hybridization buffer consisting of 6 × SSC (composition of 20 × SSC: 3 M of sodium chloride, 0.3 M of citric acid solution, pH 7.0), 5 × Denhardt's solution (composition of 100 × Denhardt's solution: 2% by mass of bovine serum albumin, 2% by mass of Ficoll, 2% by mass of polyvinylpyrrolidone), 0.5% by mass of SDS, 0.1 mg/mL of salmon sperm DNA, and 50% by volume of formamide. A washing buffer to be used for washing after incubation preferably includes a 1×SSC solution containing 0.1% by mass of SDS and more preferably a 0.1×SSC solution containing 0.1% by mass of SDS.

In (b) to (e) described above, it is preferable to use degenerate codons having a high codon usage frequency in somatic cells in which the quality improving agent for iPS cells according to the present embodiment is used. For example, in a case of using in human somatic cells, it is preferable to use codons having a high usage frequency in human cells. That is, those that are optimized as human codons are preferable. In addition, in a case of using in mouse somatic cells, it is preferable to use codons having a high usage frequency in mouse cells. That is, those that are optimized as mouse codons are preferable.

In the present specification, "H1foo protein" may refer to a fusion protein of a destabilization domain described later and the H1foo protein, or an H1foo protein region in the fusion protein.

### (Regulatory sequence)

The regulatory sequence is a nucleotide sequence that is capable of regulating at least one of the amount and the period of the existence of the H1foo protein expressed from the H1foo gene in cells when the H1foo gene is transduced into the cells. The regulation method is not particularly limited as long as it is capable of regulating any one of the amount or the period of the existence of the H1foo protein in cells. Examples of the regulatory sequence include a nucleotide sequence encoding a destabilization domain. Alternatively, examples of the regulatory sequence include a promoter sequence that regulates the transcription of the H1foo gene in response to a chemical stimulus.

In the present specification, the term "destabilization domain (DD)" refers to a domain that promotes proteasomal degradation of a fusion protein containing the destabilization domain. That is, in a case where a destabilization domain is linked to the N-terminal or C-terminal of a desired protein to form a fusion protein containing the destabilization domain, the proteasomal degradation of the fusion protein containing the desired protein is promoted.

The destabilization domain is not particularly limited as long as it promotes the degradation of a fusion protein containing the destabilization domain, and known a destabilization domain can be used. Examples of the destabilization domain include a destabilization domain derived from FKBP12 (Banaszynski et al., Cell. 2006 Sep 8; 126(5): 995-1004.) and a destabilization domain derived from ecDHFR (Iwamoto et al., Chem Biol. 2010 Sep 24; 17(9): 981-8.). Examples of the destabilization domain derived from FKBP12 include a destabilization domain having an amino acid sequence set forth in SEQ ID NO: 14, and examples of the nucleotide sequence encoding this amino acid sequence include a nucleotide sequence set forth in SEQ ID NO: 13. It is noted that a fusion protein of a desired protein and a destabilization domain may contain a polypeptide between the desired protein and the destabilization domain.

The degradation of fusion proteins containing these destabilization domains can be suppressed by a membrane-permeable low-molecular-weight compound (hereinafter, referred to as a "stabilization compound") called Shield1 (for a destabilization domain derived from FKBP12) or Guard (for a destabilization domain derived from ecDHFR). Polynucleotides encoding these destabilization domains (hereinafter, referred to as "destabilization domain genes") and stabilization compounds can be used through PreteoTuner^{™} Shield System (Clontech), PreteoTuner^{™} Guard System (Clontech), and the like.

A destabilization domain can also be said to be a degron sequence. Examples of the degron include an mTOR degron (Unite States Patent Application, Publication No. 2009/0215169), a dihydrofolate reductase (DHFR) degron (Unite States Patent Application, Publication No. 2012/0178168), PEST (PCT International Publication No. WO99/54348), a TetR degron (PCT International Publication No. WO2007/032555), and an auxin-inducible degron (AID) (PCT International Publication No. WO2010/125620), which are not limited thereto.

The destabilization domain gene may be linked to the H1foo gene so that a fusion protein of the destabilization domain and the H1foo protein is capable of being expressed. As a result, in a case where the quality improving agent for iPS cells according to the present embodiment is introduced into cells, the H1foo protein is expressed as a fusion protein fused with the destabilization domain. Therefore, the H1foo protein is rapidly degraded by the proteasome after expression. That is, in a case of expressing the H1foo protein as a fusion protein fused with a destabilization domain, it is possible to regulate the amount of the H1foo protein in cells to be reduced. Furthermore, in a case of using, as an expression vector for a fusion protein, a vector that expresses a gene in the cytoplasm without incorporating the gene into the host chromosome and disappears quickly from the cell after gene expression, it is possible to regulate the period of the existence of the H1foo protein within a certain period after the introduction of the expression vector. In this way, in a case where the H1foo protein is expressed for a certain period of time from the introduction of the nuclear reprogramming factor to the completion of the reprogramming and thereafter is regulated to be present at a relatively low level, it is possible to prepare iPS cells having high quality, with highly efficiency.

In addition, the addition of a stabilization compound also makes it possible to regulate a fusion protein containing a destabilization domain in terms of the amount and the period of the existence in cells. For example, the amount and the period of the existence of the H1foo protein may be regulated by introducing the quality improving agent for iPS cells according to the present embodiment into somatic cells together with a nuclear reprogramming factor described later, subsequently adding a stabilization compound for any period of time, and removing the stabilization compound after any period of time described above has elapsed.

In addition, the form of the polynucleotide contained in the quality improving agent for iPS cells according to the present embodiment may be such that the H1foo gene is inserted into an expression vector in a state of being capable of being expressed in cells into which the H1foo gene is introduced. The destabilization domain gene may be linked to a replication-associated protein gene so that a fusion protein of the destabilization domain and a protein necessary for the replication of the expression vector (hereinafter, referred to as a "replication-associated protein") is capable of being expressed. The "replication-associated protein gene" means a polynucleotide that encodes a replication-associated protein. A replication-associated protein expressed as a fusion protein fused with a destabilization domain is rapidly degraded by the proteasome after expression. Therefore, the replication of the expression vector is inhibited due to the decrease in the amount of the replication-associated protein. This makes it possible to indirectly regulate the period of the existence and the amount of the H1foo protein in cells.

The replication-associated protein to form a fusion protein with a destabilization domain is not particularly limited, and it may be appropriately selected depending on the kind of expression vector. For example, in a case where the expression vector is a Sendai virus vector, examples of the replication-associated protein include nucleocapsid protein (N), phosphorylated protein (P), matrix protein (M), and large protein (L). Among these, the P protein is preferable.

In a case where the expression vector contains a plurality of replication-associated protein genes, the destabilization domain gene may be linked to any two or more of the plurality of replication-associated protein genes.

In a case where the regulatory sequence includes a destabilization domain gene sequence, the regulatory sequence may be linked to the H1foo gene or at least one of the 5' terminal and the 3' terminal of the replication-associated protein gene. The regulatory sequences may be linked to any one of the 5' terminal or the 3' terminal of the protein gene or may be linked to both the 5' terminal and the 3' terminal. The linking between the regulatory sequence and the H1foo gene or the replication-associated protein gene is carried out so that no frameshift occurs (that is, in-frame) in the destabilization domain gene and the H1foo gene or the replication-associated protein gene. As an example, SEQ ID NO: 15 shows a nucleotide sequence in a case where a destabilization domain gene derived from FKBP12 is linked to the 3' terminal of the H1foo gene. The nucleotide sequence set forth in SEQ ID NO: 15 encodes a fusion protein (SEQ ID NO: 16) in which the destabilization domain gene derived from FKBP12 is added to the C-terminal of the H1foo protein. As an example, SEQ ID NO: 17 shows a nucleotide sequence in a case where a destabilization domain gene derived from FKBP12 is linked to the 5' terminal of the H1foo gene. The nucleotide sequence set forth in SEQ ID NO: 17 encodes a fusion protein (SEQ ID NO: 18) in which the destabilization domain gene derived from FKBP12 is added to the N-terminal of the H1foo protein.

The regulatory sequence may be linked to both the H1foo gene and the replication-associated protein gene.

The regulatory sequence may include a promoter sequence that regulates the transcription of the H1foo gene in response to an external stimulus. Examples of the external stimuli include a chemical substance, heat, light, pH, and osmotic pressure. A promoter that regulates the transcription of the H1foo gene in response to an external stimulus (hereinafter, referred to as an "external stimulus-responsive promoter") is positioned upstream of the H1foo gene to regulate the transcription of the H1foo gene.

The external stimulus-responsive promoter is not particularly limited, and a known promoter can be used. Examples of the external stimulus-responsive promoter include a tetracycline-responsive promoter (for example, a tetracycline-responsive element: TRE).

### (Polynucleotide)

The quality improving agent for iPS cells according to the present embodiment contains a polynucleotide having the H1foo gene and the regulatory sequence. The polynucleotide is such that the H1foo gene is inserted into an expression vector in a state capable of expressing the H1foo gene under the regulation of a regulatory sequence.

The "expression vector that is capable of expressing the H1foo gene" may be an expression vector that expresses the H1foo protein or may be an expression vector that expresses a fusion protein of the H1foo protein and the destabilization domain.

In addition to the H1foo gene and the regulatory sequence, the expression vector preferably contains, as necessary, a promoter that regulates the expression of the H1foo gene or the expression of the H1foo gene and the destabilization domain gene (hereinafter, collectively referred to as "the H1foo gene and the like"). In the expression vector, the promoter is positioned upstream of the H1foo gene and the like so that the expression of the H1foo gene and the like are regulated. However, in a case where the regulatory sequence is an external stimulus-responsive promoter, the expression vector preferably does not contain another promoter that regulates the expression of the H1foo gene.

Examples of the promoter include an SRα promoter, an SV40 early promoter, LTR of a retrovirus, a cytomegalovirus (CMV) promoter, a Rous sarcoma virus (RSV) promoter, a herpes simplex virus thymidine kinase (HSV-TK) promoter, an EF1α promoter, a metallothionein promoter, and a heat shock promoter. In addition, an enhancer of the IE gene of human CMV may be used together with the promoter. As an example, a CAG promoter (containing a cytomegalovirus enhancer, a chicken β-actin promoter, and a poly A signal site of the β-globin gene) can be used.

In addition, the stimulus-responsive promoters mentioned in the section of "Regulatory sequence" described above may be used.

In addition to the promoter, the expression vector may contain, as desired, an enhancer, a poly(A) addition signal, a marker gene, a replication origin, and a gene encoding a protein that binds to the replication origin and regulates replication. The marker gene is a gene that enables the sorting or selection of cells by introducing the marker gene into cells. Specific examples of the marker gene include a drug resistance gene, a fluorescent protein gene, a luminescent enzyme gene, and a chromogenic enzyme gene. One kind of these may be used alone, or two or more kinds thereof may be used in combination. Specific examples of the drug resistance gene include a neomycin resistance gene, a tetracycline resistance gene, a kanamycin resistance gene, a zeocin resistance gene, a hygromycin resistance gene, and a puromycin resistance gene. Specific examples of the fluorescent protein genes include a green fluorescent protein (GFP) gene, a yellow fluorescent protein (YFP) gene, and a red fluorescent protein (RFP) gene. Specific examples of the luminescent enzyme gene include a luciferase gene. Specific examples of the chromogenic enzyme gene include a β-galactosidase gene, a β-glucuronidase gene, and an alkaline phosphatase gene.

In a case where a stimulus-responsive promoter is used as the promoter, an enhancer gene or repressor gene which binds to the promoter in response to the stimulus may be contained in the expression vector. For example, in a case of a tetracycline-responsive promoter, the expression vector may contain a gene such as a reverse tetracycline-regulatable transactivator (rtTA) or a tetracycline-regulatable transactivator (rTA). This enhancer gene or repressor gene may be contained in an expression vector separately from the expression vector having the H1foo gene.

The kind of expression vector is not particularly limited, and a known expression vector can be used. Examples of the expression vector include an episomal vector, an artificial chromosome vector, a plasmid vector, and a virus vector.

The episomal vector is a vector that is capable of being autonomously replicated outside the chromosome. Specific means for using an episomal vector is disclosed in Yu et al., Science, 324, 797-801 (2009). For example, it is possible to use an episomal vector in which loxP sequences are positioned in the same direction on the 5' side and the 3' side of a vector element necessary for the replication of the episomal vector. Since the episomal vector can be autonomously replicated outside the chromosome, it can provide stable expression in host cells without being incorporated into the genome; however, it is desirable that the vector is quickly removed after once iPS cells have been established. In a case where a vector element required for the replication of the episomal vector is sandwiched between two loxP sequences, and a Cre recombinase is allowed to act thereon to excise the vector element, the autonomous replication ability of the episomal vector can be lost, and thus the vector can be dropped out of iPS cells at an early stage.

Examples of the episomal vector include a vector containing, as a vector element, a sequence necessary for autonomous replication, which is derived from EBV, SV40, or the like. Specifically, the vector element necessary for autonomous replication includes a replication origin and a gene encoding a protein that binds to the replication origin to regulate replication, and examples thereof include the replication origin oriP and the EBNA-1 gene for SV40 and the replication origin oriP and the SV40 LT gene for SV40.

Examples of the artificial chromosome vector include a yeast artificial chromosome (YAC) vector, a bacterial artificial chromosome (BAC) vector, and a P1-derived artificial chromosome (PAC) vector.

The plasmid vector is not particularly limited as long as it is a plasmid vector that is capable of being expressed in somatic cells to be introduced. In a case where the somatic cells to be introduced are mammalian cells, a general expression plasmid vector for animal cells can be used. Examples of the expression plasmid vector for animal cells include pA1-11, pXT1, pRc/CMV, pRc/RSV, and pcDNAI/Neo.

Examples of the virus vector include a retrovirus (including a lentivirus) vector, an adenovirus vector, an adeno-associated virus vector, a Sendai virus vector, a herpes virus vector, a vaccinia virus vector, a pox virus vector, a polio virus vector, a Sindbis virus vector, a rhabdovirus vector, a paramyxovirus vector, and an orthomyxovirus vector.

In the present specification, the virus vector means a vector that has a genomic nucleic acid derived from the virus and can express a transgene by incorporating the transgene into the nucleic acid.

The virus vector is preferably a Sendai virus vector. The Sendai virus is one of the viruses of the Mononegavirales, belongs to the Paramyxoviridae (Paramyxoviridae; including genera such as Paramyxovirus, Morbillivirus, Rubulavirus, and Pnemovirus), and contains an RNA of one minus strand (the antisense strand against the sense strand encoding viral proteins) as the genome. The minus strand RNA is also called a negative strand RNA. The Sendai virus vector is a chromosomally non-incorporating virus vector, and the vector is expressed in the cytoplasm. Therefore, there is no risk that the transgene is incorporated into the host chromosome. As a result, the safety is high, and the vector can be removed from the post-introduction cells after the purpose is achieved.

In addition to infectious virus particles, the Sendai virus vector includes a complex of the viral core, the viral genome, and the viral proteins or a complex consisting of the non-infectious virus particles or the like, where the complex has the ability to express a gene carried thereon, in a case of being introduced into cells. For example, a ribonucleoprotein (a virus core portion) consisting of the Sendai virus genome and the Sendai virus proteins (NP, P, and L proteins) that bind to the Sendai virus genome makes it possible to express a transgene in cells by being introduced into cells (PCT International Publication No. WO00/70055). The introduction into cells may be carried out using an appropriate transfection reagent or the like. Therefore, such a ribonucleoprotein (RNP) is also included in the Sendai virus vector.

The genome of the Sendai virus includes a nucleocapsid (NP) gene, a phospho (P) gene, a matrix (M) gene, a fusion (F) gene, a hemagglutinin/neuraminidase (HN) gene, and a large (L) gene, in order from the 3' end to the 5' end. In a case of having, among them, the NP gene, the P gene, and the L gene, the Sendai virus can sufficiently function as a vector, the genome thereof can be replicated in cells, and a gene carried thereon can be expressed. The Sendai virus has a minus strand RNA in the genome thereof, and thus contrary to normal cases, the 3' side of the genome corresponds to the upstream side, and the 5' side thereof corresponds to the downstream side.

The accession number of the base sequence database (GenBank) of each of the above genes of the Sendai virus can be identified by referencing, for example, for the NP gene, M29343, M30202, M30203, M30204, M51331, M55565, M69046, and X17218; for the P gene, M30202, M30203 M30204, M55565, M69046, X00583, X17007, and X17008; for the M gene, D11446, K02742, M30202, M30203, M30204, M69046, U31956, X00584, and X53056; for the F gene, D00152, D11446, D17334, D17335, M30202, M30203, M30204, M69046, X00152, and X02131; for the HN gene, D26475, M12397, M30202, M30203, M30204, M69046, X00586, X02808, and X56131; and for the L gene, D00053, M30202, M30203, M30204, M69040, X00587, and X58886.

However, a plurality of Sendai virus strains are known, and thus depending on the strain, there is also a gene consisting of a sequence other than those provided as exemplary examples above. A Sendai virus vector having a viral gene derived from any one of these genes is also useful as the Sendai virus vector. For example, the Sendai virus vector can contain a base sequence having 90% or more of a sequence identity and preferably 95% or more, 96% or more, 97% or more, 98% or more, or 99% or more of sequence identity with the coding sequence of any one of the above viral genes. In addition, the Sendai virus vector can contain a base sequence encoding an amino acid sequence that has, for example, 90% or more of a sequence identity and preferably 95% or more, 96% or more, 97% or more, 98% or more, or 99% or more of sequence identity with the amino acid sequence encoded by the coding sequence of any one of the above viral genes. In addition, in the amino acid sequence encoded by the coding sequence of any of the above viral genes, the Sendai virus vector can contain a base sequence encoding an amino acid sequence in which for example, amino acids of 10 or less amino acids and preferably 9 or less amino acids, 8 or less amino acids, 7 or less amino acids, 6 or less amino acids, 5 or less amino acids, 4 or less amino acids, 3 or less amino acids, 2 or less amino acids, or one amino acid have undergone substitution, insertion, deletion, and/or addition, where the amino acid sequence is a polypeptides that retains the function of each gene product.

Sequences referenced by database accession numbers, such as base sequences and amino acid sequences described in the present specification are referenced as sequences based on the filing date of the present application and are identified as sequences based on the timing of the filing date of the present application. The sequence at each timing can be identified by referencing the revision history of the database.

The Sendai virus vector that is used in the present embodiment may be a derivative thereof. Examples of the derivative of the Sendai virus vector include a virus in which viral genes have been modified so that the gene introduction ability by the Sendai virus is not impaired, and a virus that has been chemically modified.

The Sendai virus may be derived from a natural strain, a wild strain, a mutant strain, a laboratory passaged strain, an artificially constructed strain, or the like. For example, the Z strain can be included (Medical Journal of Osaka University Vol. 6, No. 1, March 1955, p1-15). In other words, the virus may be a virus vector having a structure similar to that of a virus isolated from nature or may be a virus artificially modified by genetic recombination, as long as a target function can be achieved. For example, the virus may be such that there is a mutation or a deletion in any one of genes of a wild-type virus. In addition, it is also possible to use an incomplete virus such as DI particles (J. Virol. 68: 8413-8417, 1994). For example, it is possible to suitably use a virus having mutation or deletion in at least one gene encoding a viral envelope protein or coat protein. Such a virus vector is, for example, a virus vector that makes it possible to replicate the genome of the virus in infected cells but does not make it possible to form infectious virus particles. Such a transmissibility-defective virus vector is highly safe since there are no worries about spreading the infection to the surroundings. For example, it is possible to use a virus vector that does not contain at least one gene encoding an envelope protein such as F and/or HN or a spike protein, or a combination thereof (PCT International Publication No. WO00/70055, PCT International Publication No. WO00/70070, or Li, H.-O. et al., J. Virol. 74 (14) 6564-6569 (2000)). The genome can be amplified in infected cells in a case where the genomic RNA encodes the proteins required for genome replication (for example, the NP, P, and L proteins). In order to produce a defective virus, for example, a deleted gene product or a protein capable of complementing the deleted gene product is exogenously supplied in virus-productive cell cells (PCT International Publication No. WO00/70055, PCT International Publication No. WO00/70070, or Li, H.-O. et al., J. Virol. 74 (14) 6564-6569 (2000)). In a case where a virus vector is recovered as an RNP (for example, an RNP consisting of N, L, and P proteins, and a genomic RNA), a vector can be produced without complementing the envelope protein.

The suitable Sendai virus vector may be, for example, an F gene-deleted Sendai virus vector (for example, a Z strain), in which mutations of G69E, T116A, and A183S are present in the M protein, mutations of A262T, G264, and K461G are present in the HN protein, a mutation of L511F is present in the P protein, and mutations of N1197S and K1795E are present in the L protein, or it may be a vector obtained by further introducing a mutation of TS 7, TS 12, TS 13, TS 14, or TS 15 into this vector. Specific examples thereof include SeV18+/TSΔF (PCT International Publication No. WO2010/008054 and PCT International Publication No. WO2003/025570), SeV(PM)/TSΔF, and a vector obtained by further introducing a mutation of TS 7, TS 12, TS 13, TS 14, or TS 15, which are not limited thereto.

"TSΔF" has mutations of G69E, T116A, and A183S in the M protein, mutations of A262T, G264R, and K461G in the HN protein, a mutation of L511F in the P protein, and mutations of N1197S and K1795E in the L protein, which refers to that the F gene is deleted.

Suitable examples of the Sendai virus vector include a vector in which a degron sequence is added to the P protein so that the vector can be easily removed from infected cells (PCT International Publication No. WO2016/125364). Examples of the degron include an mTOR degron (Unite States Patent Application, Publication No. 2009/0215169), a dihydrofolate reductase (DHFR) degron (Unite States Patent Application, Publication No. 2012/0178168), PEST (PCT International Publication No. WO99/54348), a TetR degron (PCT International Publication No. WO2007/032555), and an auxin-inducible degron (AID) (PCT International Publication No. WO2010/125620), which are not limited thereto. For example, a vector in which a DD-tag, which is one kind of the mTOR degron, is added to the C-terminal of the P protein is suitably used.

A recombinant Sendai virus vector having a polynucleotide to be introduced (the H1foo gene, or the H1foo gene and a regulatory sequence) can be reconstituted using a known method.

Specifically, it can be produced by (a) a step of transcribing a cDNA encoding the Sendai virus genomic RNA (the minus strand) or the complementary strand thereof (the plus strand) in cells expressing viral proteins (N, P, and L) required for virus particle formation, and (b) a step of recovering the culture supernatant containing the generated virus. The viral proteins required for particle formation may be expressed from transcribed viral genomic RNA or may be supplied in trans from sources other than genomic RNA. For example, the N, P, and L proteins can be supplied by introducing an expression plasmid encoding the N, P, and L proteins into cells. In a case where a viral gene necessary for particle formation is deleted in the genomic RNA, the viral gene can be separately expressed in virus-productive cell cells to complement the particle formation. In order to express viral proteins or an RNA genome in cells, a vector, in which a DNA encoding the viral proteins or the genomic RNA is linked downstream of an appropriate promoter that functions in host cells, is introduced into host cells. The transcribed genomic RNA is replicated in the presence of the viral proteins to form infectious virus particles. In a case of producing a defective virus in which a gene such as an envelope protein is deleted, it is possible to express, in virus-productive cell cells, another viral protein that can complement the defective protein or the function of the defective protein.

In addition, the Sendai virus can be produced using the following known methods (PCT International Publication No. WO97/16539; PCT International Publication No. WO97/16538; PCT International Publication No. WO00/70055; PCT International Publication No. WO00/70070; PCT International Publication No. WO01/18223; PCT International Publication No. WO03/025570; PCT International Publication No. WO2005/071092; PCT International Publication No. WO2006/137517; PCT International Publication No. WO2007/083644; PCT International Publication No. WO2008/007581; Hasan, M. K. et al., J. Gen. Virol. 78: 2813-2820, 1997, Kato, A. et al., 1997, EMBO J. 16: 578-587, and Yu, D. et al., 1997, Genes Cells 2: 457-466; Durbin, A. P. et al., 1997, Virology 235: 323-332; Whelan, S. P. et al., 1995, Proc. Natl. Acad. Sci. USA 92: 8388-8392; Schnell. M. J. et al., 1994, EMBO J. 13: 4195-4203; Radecke, F. et al., 1995, EMBO J. 14: 5773-5784; Lawson, N. D. et al., Proc. Natl. Acad. Sci. USA 92: 4477-4481; Garcin, D. et al., 1995, EMBO J. 14: 6087-6094; Kato, A. et al., 1996, Genes Cells 1: 569-579; Baron, M. D., and Barrett, T., 1997, J. Virol. 71: 1265-1271; Bridgen, A. and Elliott, R. M., 1996, Proc. Natl. Acad. Sci. USA 93: 15400-15404; Tokusumi, T. et al. Virus Res. 2002: 86; 33-38, and Li, H.-O. et al., J. Virol. 2000: 74; 6564-6569).

In a case where the Sendai virus vector is used, the H1foo gene and the like may be positioned at any position of the Sendai virus genomic RNA (the minus strand) as long as each gene of the Sendai virus is not disrupted. For example, the H1foo gene and the like may be positioned on the 3' side from the NP gene, may be positioned between the NP gene and the P gene, may be positioned between the P gene and the M gene, may be positioned between the M gene and the F gene, may be positioned between the F gene and the HN gene (between the M gene and the HN gene in a case where the F gene is absent), may be between the HN gene and the L gene, or may be on the 5' side from the L gene. In order to increase the expression level of the H1foo gene and the like, it is preferable that the H1foo gene and the like are positioned closer to the 3' side. For example, in a case where the H1foo gene and the like are positioned closest to the 3' side (for example, on the 3' side from the NP gene) on the Sendai virus genomic RNA (the minus strand), the expression levels of the H1foo gene and the like increase most. In a case where the Sendai virus vector is used, it is preferable that the H1foo gene and the like are positioned on the 3' side from all the Sendai virus genes. That is, it is preferable that the H1foo gene and the like are positioned closet to the 3' side. This makes it easy to suitably maintain the balance between the expression level of the H1foo protein and the level of the degradation of the H1foo protein promoted by the destabilization domain. As a result, it is possible to prepare iPS cells having a higher quality, with highly efficiency. In addition, in a case where cells to be introduced are infected with the Sendai virus, it is possible to prepare iPS cells having a higher quality, with highly efficiency.

In a case where a virus vector is used as an expression vector, virus particles obtained by using packaging cells may be used. The packaging cell is a cell into which a gene encoding a viral structural protein has been introduced, and thus in a case where a recombinant virus vector into which a target gene has been incorporated is introduced into the packaging cell, recombinant virus particles into which the target gene has been incorporated are produced. The packaging cell is not particularly limited and can be appropriately selected depending on the intended purpose. Examples thereof include a packaging cell based on the HEK293 cell derived from the human kidney or the NIH3T3 cell derived from the mouse fibroblast; a PLAT-E cell designed to express an ecotropic virus-derived envelope glycoprotein; a PLAT-A cell designed to express an amphotropic virus-derived envelope glycoprotein; and a PLAT-GP cell designed to express a vesicular stomatitis virus-derived envelope glycoprotein. In a case where a cell to be introduced with a virus vector is a human somatic cell, the packaging cell is preferably a PLAT-A cell, a PLAT-GP cell, or the like in terms of host tropism. A method for introducing a virus vector into packaging cells is not particularly limited and can be appropriately selected depending on the intended purpose. Examples thereof include a lipofection method, an electroporation method, and a calcium phosphate method.

The expression vector may contain a nuclear reprogramming factor described later. In a case where the expression vector contains a nuclear reprogramming factor, the nuclear reprogramming factor may be one kind or may be two or more kinds. In a case where the expression vector has a nuclear reprogramming factor together with the H1foo gene and the like, a base sequence encoding a self-cleavable peptide such as a 2A peptide or an internal ribozyme entry site (IRES) sequence may be interposed between the H1foo gene and the like and the nuclear reprogramming factor. In a case where this sequence is interposed therebetween, a plurality of proteins can be independently expressed from one promoter.

In a preferred aspect, the quality improving agent according to the present embodiment is a Sendai virus vector that carries a polynucleotide encoding a fusion protein of the H1foo protein and the destabilization domain in a form in a state capable of being expressed. In a more preferred aspect, the quality improving agent according to the present embodiment is a Sendai virus vector that carries a polynucleotide encoding a fusion protein of the H1foo protein and a destabilization domain derived from FKBP12 in a form in a state capable of being expressed. Specific examples of the fusion protein containing H1FOO include a protein containing the amino acid sequence set forth in SEQ ID NO: 16 or SEQ ID NO: 18. Specific examples of the polynucleotide encoding this protein include a polynucleotide containing the nucleotide sequence set forth in SEQ ID NO: 15 or SEQ ID NO: 17.

### <Second embodiment>

In one embodiment, the present invention provides a quality improving agent for iPS cells, containing a fusion protein of an H1foo protein and a destabilization domain. The destabilization domain is a domain that induces proteasomal degradation of the fusion protein.

### (H1foo protein)

The H1foo protein is a protein generated by transcription and translation from the H1foo gene described in "<First embodiment>" described above, and it has the H1foo activity. The biological species from which the H1foo protein is derived is not particularly limited and can be appropriately selected depending on the intended purpose. Examples thereof include any mammals such as a human, a mouse, a rat, a cow, a sheep, a horse, and a monkey. An amino acid sequence of the human H1foo protein is exemplified in SEQ ID NO: 2. In addition, an amino acid sequence of the mouse H1foo protein is an exemplary example in SEQ ID NO: 4.

The H1foo protein is not limited to a wild-type H1foo protein and may include those having a mutation (any one of deletion, substitution, insertion, and addition, or a combination thereof). The number of mutations is not particularly limited as long as a protein having the H1foo activity is used.

Examples of the H1foo protein include the following (a) to (c).
(a) A wild-type H1foo protein (for example, a polypeptide consisting of an amino acid sequence set forth in SEQ ID NO: 2 or SEQ ID NO: 4)
(b) A protein consisting of an amino acid sequence, in which one or a plurality of amino acids are mutated in an amino acid sequence of a wild-type H1foo protein (for example, an amino acid sequence set forth in SEQ ID NO: 2 or 4), and has the H1foo activity
(c) A protein consisting of an amino acid sequence having 70% or more of sequence identity with an amino acid sequence of a wild-type H1foo protein (for example, an amino acid sequence set forth in SEQ ID NO: 2 or 4), and has the H1foo activity

In (b) described above, "mutation" may be any one of deletion, substitution, addition, and insertion or may be a combination thereof.

In (b) described above, "a plurality of amino acids" is not particularly limited as long as the resultant protein has the H1foo activity. However, examples thereof include 2 to 30 amino acids, where 2 to 20 amino acids are preferable, 2 to 10 amino acids are more preferable, 2 to 5 amino acids are still more preferable, and 2 or 3 amino acids are particularly preferable.

In (c) described above, the sequence identity is not particularly limited as long as it is 70% or more. However, it is preferably 80% or more, more preferably 85% or more, and still more preferably 90% or more, 95% or more, 96% or more, 97% or more, 98% or more, or 99% or more.

### (Destabilization domain)

The destabilization domain is the same as those described in "<First embodiment>" described above, and examples thereof include the same ones as those exemplified in "<First embodiment>" described above.

### (Fusion protein)

The fusion protein of the H1foo protein and the destabilization domain is such that the destabilization domain is linked to at least one of the N-terminal and the C-terminal of the H1foo protein. The destabilization domain may be linked to any one of the N-terminal and the C-terminal of the H1foo protein or may be linked to both the N-terminal and the C-terminal.

The fusion protein of the H1foo protein and the destabilization domain can be produced by using an expression vector containing a polynucleotide in which a destabilization domain gene is linked to at least one of the 5' terminal and the 3' terminal of the H1foo gene. The fusion protein can be obtained by introducing the expression vector into appropriate cells, culturing the cells, and isolating and purifying a fusion protein from the culture supernatant or the cultured bacteria or cells.

The fusion protein of the H1foo protein and the destabilization domain may further have a protein transduction domain (PTD). As the PTD, those using cell translocation domains of proteins, such as AntP derived from drosophila, TAT derived from HIV, and VP22 derived from HSV have been developed. In a case where a PTD is provided, a fusion protein can be introduced into cells without using a protein introduction reagent.

Due to having the destabilization domain, the fusion protein of the H1foo protein and the destabilization domain is rapidly degraded by the proteasome in a case of being introduced into somatic cells. It is noted that the addition of a stabilization compound makes it possible to regulate a fusion protein containing a destabilization domain in terms of the amount and the periodof the existence in cells. As a result, it is possible to regulate the amount and the period of the existence of the H1foo protein in cells. In a case of introducing the quality improving agent for iPS cells according to the present embodiment into somatic cells together with a nuclear reprogramming factor described later and subsequently regulating the fusion protein of the H1foo protein and the destabilization domain to be present in the cells, for example, only for any period of time immediately after introduction, it is possible to produce iPS cells having high quality.

In the quality improving agent for iPS cells and the method of producing iPS cells of the present invention, instead of the polynucleotide having the H1foo gene and the regulatory sequence, it is possible to use a substance that is introduced into somatic cells together with a nuclear reprogramming factor and has a function of suppressing the innate immune response in the cells at the same time as the time when cell reprogramming is started. The suppression of the innate immune response specifically means the suppression of the expression of innate immune response markers such as IFIT1 and IFNA and more specifically means that the expression of FKBP1A in the cells is increased by 2 times or more, preferably 2 to 20 times, and still more preferably 5 to 15 times as compared with a case where only the nuclear reprogramming factor is introduced. Here, the time when cell reprogramming is started refers to the 2nd day to the 15th day and preferably the 3rd day to the 6th day from the introduction of the nuclear reprogramming factor. In a case of carrying out such a reprogramming step, not only the efficiency of the iPS cell establishment increases, but also a large number of iPS cells having high quality as iPS cells, such as high pluripotency, are included in the produced iPS cell population, which is also effective as a method of producing heterologous iPS cells, but it has a particularly large effect in a case where treating in bulk without establishing a cell line in a case where producing autologous iPS cells. However, this is particularly effective in a case of being handled in bulk without establishing a cell line at the time of producing autologous iPS cells.

A substance that has the function of suppressing that innate immune response is not particularly limited as long as it is capable of suppressing the expression of innate immune response markers. Examples of the substance having the function of suppressing the innate immune response include an siRNA or antisense RNA against an innate immune response marker gene, a transcription inhibitor of an innate immune response marker gene, FKBP1A, an FKBP1A gene, and a transcription promoting factor of FKBP1A, which are not limited thereto.

### [Method of producing iPS cells]

### <First embodiment>

In one embodiment, the present invention provides a method of producing iPS cells. A step of introducing a nuclear reprogramming factor and the quality improving agent for iPS cells according to the above embodiment into somatic cells is included.

### <<Introduction step>>

The method of producing iPS cells according to the present embodiment provides a method of producing iPS cells. A step of introducing a nuclear reprogramming factor and the quality improving agent for iPS cells according to the above embodiment into somatic cells is included.

### (Quality improving agent for iPS cells)

The quality improving agent for iPS cells may be any one of those according to the first embodiment and the second embodiment described in "[Quality improving agent for iPS cells]" described above. In addition, the quality improving agents for iPS cells according to both the first embodiment and the second embodiment may be used in combination.

### (Nuclear reprogramming factor)

In the present specification, the term "nuclear reprogramming factor" means a substance (a group) that can induce iPS cells from somatic cells in a case of being introduced into the somatic cells. The nuclear reprogramming factor is not particularly limited as long as it is a substance (a group) that can induce iPS cells from somatic cells. The nuclear reprogramming factor may be any substance such as a gene (including a form incorporated into an expression vector) or a gene product thereof, or a low-molecular-weight compound. The term "gene product" means an mRNA transcribed from a gene and a protein translated from the transcribed mRNA. The gene product to be used as a nuclear reprogramming factor may be an mRNA, may be a protein, or may be both an mRNA and a protein. A gene that is a nuclear reprogramming factor means a polynucleotide that encodes a protein that is a nuclear reprogramming factor.

In a case where the nuclear reprogramming factor is a gene or a gene product thereof, examples thereof include at least one selected from the group consisting of a gene of an Oct gene family, a gene of a Sox gene family, a gene of a Klf gene family, a gene of a Myc gene family, a gene of a Lin gene family, and a Nanog gene, as well as gene products thereof (PCT International Publication No. WO2007/69666; Japanese Patent No. 5696282; and Science, 2007, 318: 1917-1920). Among these, preferred examples thereof include at least one selected from the group consisting of a gene of an Oct gene family, a gene of a Sox gene family, a gene of a Klf gene family, and a gene of a Myc gene family, as well as gene products thereof.

Specific examples of these families of genes and combinations are listed below. It is noted that a case where a gene product is used is also included although only the name of the gene is described later.
(a) One nuclear reprogramming factor consisting of a gene of the Oct gene family;
(b) A combination of two nuclear reprogramming factors consisting of a gene of the Oct gene family and a gene of the Sox gene family;
(c) A combination of two nuclear reprogramming factors consisting of a gene of the Oct gene family and a gene of the Klf gene family;
(d) A combination of two nuclear reprogramming factors consisting of a gene of the Oct gene family and a Nanog gene;
(e) A combination of three nuclear reprogramming factors consisting of a gene of the Oct gene family, a gene of the Sox gene family, and a gene of the Klf gene family;
(f) A combination of three nuclear reprogramming factors consisting of a gene of the Oct gene family, a gene of the Klf gene family, and a gene of the Myc gene family;
(g) A combination of four nuclear reprogramming factors consisting of a gene of the Oct gene family, a gene of the Sox gene family, a gene of the Klf gene family, and a gene of the Myc gene family; and
(h) A combination of four nuclear reprogramming factors consisting of a gene of the Oct gene family, a gene of the Sox gene family, a gene of the Lin gene family, and a Nanog gene.

More specific examples thereof include the following combinations, which are not limited thereto. In the following combinations, a Sox2 gene can be replaced with a Sox1 gene, a Sox3 gene, a Sox15 gene, a Sox17 gene, or a Sox18 gene. A Klf4 gene can be replaced with a Klf1 gene, a Klf2 gene, or a Klf5 gene. A c-Myc gene can be replaced with a T58A (active-type mutant) gene, an N-Myc gene, or an L-Myc gene.
(1) An Oct3/4 gene, a Klf4 gene, and a c-Myc gene
(2) An Oct3/4 gene, a Sox2 gene, a Klf4 gene, and a c-Myc gene
(3) An Oct3/4 gene, a Sox2 gene, a Klf4 gene, a c-Myc gene, an Fbx15 gene, a Nanog gene, an Eras gene, an ECAT15-2 gene, a Tell gene, and β-catenin (an active-type mutant S33Y)
(4) An Oct3/4 gene, a Sox2 gene, a Klf4 gene, a c-Myc gene, an hTERT gene, and an SV40 Large T antigen (hereinafter, referred to as SV40LT) gene
(5) An Oct3/4 gene, a Sox2 gene, a Klf4 gene, a c-Myc gene, an hTERT gene, and an HPV16 E6 gene
(6) An Oct3/4 gene, a Sox2 gene, a Klf4 gene, a c-Myc gene, an hTERT gene, and an HPV16 E7 gene
(7) An Oct3/4 gene, a Sox2 gene, a Klf4 gene, a c-Myc gene, an hTERT gene, an HPV6 E6 gene, and an HPV16 E7 gene
(8) An Oct3/4 gene, a Sox2 gene, a Klf4 gene, a c-Myc gene, an hTERT gene, and a Bmil gene
   (For the combinations of (1) to (8) described above, see PCT International Publication No. WO2007/069666 (however, for the replacement of a Sox2 gene with a Sox18 gene, the replacement of a Klf4 gene with a Klf1 gene or a Klf5 gene in the combination of (2) described above, see Nature Biotechnology, 26, 101-106 (2008)). For the combination of "an Oct3/4 gene, a Sox2 gene, a Klf4 gene, and a c-Myc gene", see also Cell, 126, 663-676 (2006), Cell, 131, 861-872 (2007), and the like. For the combination of "an Oct3/4 gene, a Sox2 gene, a Klf2 (or Klf5) gene, and a c-Myc gene", see also Nat. Cell Biol., 11, 197-203 (2009). For the combination of "an Oct3/4 gene, a Sox2 gene, a Klf4 gene, a c-Myc gene, an hTERT gene, and an SV40LT gene", see also Nature, 451, 141-146 (2008)
(9) An Oct3/4 gene, a Sox2 gene, and a Klf4 gene (see Nature Biotechnology, 26, 101-106 (2008))
(10) An Oct3/4 gene, a Sox2 gene, a Nanog gene, and a Lin28 gene (see Science, 318, 1917-1920 (2007))
(11) An Oct3/4 gene, a Sox2 gene, a Nanog gene, a Lin28 gene, an hTERT gene, and an SV40LT gene (see Stem Cells, 26, 1998-2005 (2008))
(12) An Oct3/4 gene, a Sox2 gene, a Klf4 gene, a c-Myc gene, a Nanog gene, and a Lin28 gene (see Cell Research (2008) 600-603)
(13) An Oct3/4 gene, a Sox2 gene, a Klf4 gene, a c-Myc gene, and an SV40LT gene (see also Stem Cells, 26, 1998-2005 (2008))
(14) An Oct3/4 gene and a Klf4 gene (see Nature 454:646-650 (2008), Cell Stem Cell, 2: 525-528 (2008))
(15) An Oct3/4 gene and a c-Myc gene (see Nature 454: 646-650 (2008))
(16) An Oct3/4 gene and a Sox2 gene (see Nature, 451, 141-146 (2008), PCT International Publication No. WO2008/118820)
(17) An Oct3/4 gene, a Sox2 gene, and a Nanog gene (see PCT International Publication No. WO2008/118820)
(18) An Oct3/4 gene, a Sox2 gene, and a Lin28 gene (see PCT International Publication No. WO2008/118820)
(19) An Oct3/4 gene, a Sox2 gene, a c-Myc gene, and an Esrrb gene (an Esrrb gene can be replaced with an Esrrg gene, see Nat. Cell Biol., 11, 197-203 (2009))
(20) An Oct3/4 gene, a Sox2 gene, and an Esrrb gene (see Nat. Cell Biol., 11, 197-203 (2009))
(21) An Oct3/4 gene, a Klf4 gene, and an L-Myc gene
(22) An Oct3/4 gene and a Nanog gene
(23) An Oct3/4 gene
(24) An Oct3/4 gene, a Klf4 gene, a c-Myc gene, a Sox2 gene, a Nanog gene, a Lin28 gene, and an SV40LT gene (see Science, 324: 797-801 (2009))

In the combinations (1) to (24) described above, another Oct gene family member gene (for example, Oct1A, Oct6, or the like) can be used instead of the Oct3/4 gene. Instead of the Sox2 gene (or the Sox1 gene, the Sox3 gene, the Sox15 gene, the Sox17 gene, or the Sox18 gene), another Sox gene family member gene (for example, a Sox7 gene) can be used. Instead of the Lin28 gene, another Lin gene family member gene (for example, a Lin28b gene) can also be used.

A combination that does not correspond to the combinations (1) to (24) described above but includes all of the constitutional elements in any combinations of (1) to (24) and further includes any other substance can be also included in the category of the "nuclear reprogramming factor" in the present invention. Under conditions in which a somatic cell to be targeted for nuclear reprogramming endogenously expresses a part of the constitutional elements in any one of the combinations (1) to (24) described above at a level sufficient for nuclear reprogramming, a combination of only the remaining components excluding the part of constitutional elements may also be included in the category of the "nuclear reprogramming factor" in the present invention.

In addition to the above-described nuclear reprogramming factor, one or more nuclear reprogramming factors selected from the group consisting of an Fbx15 gene, an Eras gene, an ECAT15-2 gene, a Tcl1 gene, and a β-catenin gene may be combined, and/or one or more nuclear reprogramming factors selected from the group consisting of an ECAT1 gene, an Esg1 gene, a Dnmt3L gene, an ECAT8 gene, a Gdf3 gene, an Mybl2 gene, an ECAT15-1 gene, an Fthl17 gene, a Sall4 gene, a Rex1 gene, a UTF1 gene, a Stella gene, a Stat3 gene, and a Grb2 gene can also be combined. These combinations are specifically described in PCT International Publication No. WO2007/69666.

Preferred examples of the nuclear reprogramming factor include at least one selected from the group consisting of an Oct3/4 gene, a Sox2 gene, a Klf4 gene, a c-Myc gene (or al L-Myc gene), a Lin28 gene, a Nanog gene, and gene products of these genes. A combination of two or more selected from the group consisting of an Oct3/4 gene, a Sox2 gene, a Klf4 gene, a c-Myc gene (or an L-Myc gene), a Lin28 gene, a Nanog gene, and gene products thereof is preferable, and a combination of three or more thereof is preferable. Among these, examples of the combination of nuclear reprogramming factors that are preferable at least to be introduced include (1) an Oct3/4 gene or a gene product thereof, a Sox2 gene or a gene product thereof, and a Klf4 gene or a gene product thereof, (2) an Oct3/4 gene or a gene product thereof, a Sox2 gene or a gene product thereof, a Klf4 gene or a gene product thereof, and a c-Myc gene or a gene product thereof, and (3) an Oct3/4 gene or a gene product thereof, a Sox2 gene or a gene product thereof, a Klf4 gene or a gene product thereof, and an L-Myc gene or a gene product thereof. Among them, a combination of an Oct3/4 gene or a gene product thereof, a Sox2 gene or a gene product thereof, and a Klf4 gene or a gene product thereof, and an Oct3/4 gene or a gene product thereof, a Sox2 gene or a gene product thereof, a Klf4 gene or a gene product thereof, and an L-Myc gene or a gene product thereof are preferable. Among them, a combination of an Oct3/4 gene, a Sox2 gene, and a Klf4 gene, and a combination of an Oct3/4 gene, a Sox2 gene, a Klf4 gene, and an L-Myc gene are more preferable.

In a case where the nuclear reprogramming factor is a gene or a gene product thereof, the biological species from which such a gene is derived is not particularly limited and can be appropriately selected depending on the intended purpose, and examples thereof include any mammals such as a human, a mouse, a rat, a cow, a sheep, a horse, and a monkey.

The cDNA sequence information of each of the above-described nuclear reprogramming factors can be obtained from a publicly known database. For example, the accession numbers in GenBank, which are described in PCT International Publication No. WO2007/069666, may be referenced. The Nanog gene is described under the name "ECAT4" in PCT International Publication No. WO2007/069666.

Mouse and human cDNA sequence information of four particularly preferred genes (Oct3/4 gene, a Sox2 gene, a Klf4 gene, an L-Myc gene) among the above-described nuclear reprogramming factors are described below.

| Gene name | Mouse | Human |
|---|---|---|
| Oct3/4 | NM_013633 | NM_002701 |
| Sox2 | NM_011443 | NM_003106 |
| Klf4 | NM_010637 | NM_004235 |
| L-Myc | NM_008506 | NM_001033081 |

The cDNA sequence of the human Oct3/4 gene registered in the GenBank accession number is set forth in SEQ ID NO:5, and the amino acid sequence of the human Oct3/4 protein is set forth in SEQ ID NO: 6. The cDNA sequence of the human Sox2 gene is set forth in SEQ ID NO: 7, and the amino acid sequence of the human Sox2 protein is set forth in SEQ ID NO: 8. The cDNA sequence of the human Klf4 gene is set forth in SEQ ID NO: 9 and the amino acid sequence of the human Klf4 protein is set forth in SEQ ID NO: 10. The cDNA sequence of the human L-Myc gene is set forth in SEQ ID NO: 11, and the amino acid sequence of the human L-Myc protein is set forth in SEQ ID NO: 12.

In addition, mouse and human cDNA sequence information of genes of which the GenBank accession numbers are not described in PCT International Publication No. WO2007/069666 among the nuclear reprogramming factors described above are described below.

| Gene name | Mouse | Human |
|---|---|---|
| Lin28 | NM_145833 | NM_024674 |
| Lin28b | NM_001031772 | NM_001004317 |
| Esrrb | NM_011934 | NM_004452 |
| Esrrg | NM_011935 | NM_001438 |

Based on the above-described cDNA sequence information or the sequence information registered in a known database, the cDNA of each of the above-described nuclear reprogramming factors can be easily isolated from cells of an organism from which the sequence is derived, by using a known method such as PCR.

In a case where the nuclear reprogramming factor is each of the above genes or an mRNA thereof, the nucleotide sequence thereof is not limited to that of the wild-type gene, and a mutation may be contained as long as the nuclear reprogramming action is provided. Each nucleotide sequence of each of the above genes may be a protein-coding sequence only or may contain a portion other than the protein-coding sequence (for example, intron, 5'UTR, 3'UTR, or the like).

In a case where the nuclear reprogramming factor is a protein encoded by each of the above genes, the amino acid sequence thereof is not limited to the amino acid sequence of the wild-type protein, and a mutation may be contained as long as the nuclear reprogramming action is provided.

In the present specification, the term "nuclear reprogramming action" means an action that induces iPS cells from somatic cells in a case of being introduced into the somatic cells.

In a case where the nuclear reprogramming factor is a gene or mRNA, examples of the nuclear reprogramming factor include the following (a) to (g).
(a) The wild-type gene or wild-type mRNA that is provided as an exemplary example of the nuclear reprogramming factor
(b) A polynucleotide that consists of a nucleotide sequence encoding the wild-type protein provided as an exemplary example of the nuclear reprogramming factor
(c) A polynucleotide that encodes a protein consisting of an amino acid sequence, in which one or a plurality of amino acids are mutated in the wild-type protein provided as an exemplary example of the nuclear reprogramming factor, and having the nuclear reprogramming action
(d) A polynucleotide that encodes a protein consisting of an amino acid sequence, which has 70% or more of sequence identity with an amino acid sequence of the wild-type protein provided as an exemplary example of the nuclear reprogramming factor, and having the nuclear reprogramming action
(e) A polynucleotide that encodes a protein consisting of a nucleotide sequence, in which one or a plurality of nucleotides are mutated in a nucleotide sequence of the wild-type gene provided as an exemplary example of the nuclear reprogramming factor, and having the nuclear reprogramming action
(f) A polynucleotide that consists of a nucleotide sequence, which has 70% or more of sequence identity with a nucleotide sequence of the wild-type gene provided as an exemplary example of the nuclear reprogramming factor, and encodes a protein having the nuclear reprogramming action
(g) A polynucleotide that hybridizes under stringent conditions with a nucleotide sequence of the wild-type gene provided as an exemplary example of the nuclear reprogramming factor and encodes a protein having the nuclear reprogramming action

In (c) and (e) described above, "mutation" may be any one of deletion, substitution, addition, and insertion or may be a combination thereof.

In (c) described above, "a plurality of amino acids" is not particularly limited as long as the resultant protein has the nuclear reprogramming activity. However, examples thereof include 2 to 30 amino acids, where 2 to 20 amino acids are preferable, 2 to 10 amino acids are more preferable, 2 to 5 amino acids are still more preferable, and 2 or 3 amino acids are particularly preferable.

In (e) described above, "a plurality of nucleotides" is not particularly limited as long as the resultant polynucleotide encodes a protein having the nuclear reprogramming action. However, examples thereof include 2 to 60 nucleotides, where 2 to 50 nucleotides are preferable, 2 to 40 nucleotides or 2 to 30 nucleotides are more preferable, 2 to 20 nucleotides or 2 to 10 nucleotides are still more preferable, and 2 to 5 nucleotides or 2 or 3 nucleotides are particularly preferable.

In (d) and (f) described above, the sequence identity is not particularly limited as long as it is 70% or more. However, it is preferably 80% or more, more preferably 85% or more, and still more preferably 90% or more, 95% or more, 96% or more, 97% or more, 98% or more, or 99% or more.

In (g) described above, examples of the "stringent conditions" include the same conditions as the conditions exemplified in "[Quality improving agent for iPS cells]", <First embodiment>, and (Hlfoo gene) described above.

In (b) to (e) described above, it is preferable to use degenerate codons having a high codon usage frequency in somatic cells in which the quality improving agent for iPS cells according to the present embodiment is used. For example, in a case of using in human somatic cells, it is preferable to use codons having a high usage frequency in human cells. That is, those that are optimized as human codons are preferable. In addition, in a case of using in mouse somatic cells, it is preferable to use codons having a high usage frequency in mouse cells. That is, those that are optimized as mouse codons are preferable.

In a case where the nuclear reprogramming factor is a gene, the gene is preferably incorporated into an expression vector. Examples of the expression vector include those mentioned in the sections of "[Quality improving agent for iPS cells] and (Polynucleotide)" described above. Among them, a Sendai virus vector is preferable as the expression vector. In a case where an expression vector is used, only one nuclear reprogramming factor may be incorporated into one expression vector, or two or more nuclear reprogramming factors may be incorporated into one vector. In a case where the expression vector contains two or more kinds of nuclear reprogramming factors, a base sequence encoding a self-cleavable peptide such as a 2A peptide or an IRES sequence may be interposed between two or more genes which are nuclear reprogramming factors.

In a case where the nuclear reprogramming factor is a protein, examples of the nuclear reprogramming factor include the following (A) to (C).
(A) The wild-type protein that is provided as an exemplary example of the nuclear reprogramming factor
(B) A protein that consists of an amino acid sequence, in which one or a plurality of amino acids are mutated in an amino acid sequence of the wild-type protein provided as an exemplary example of the nuclear reprogramming factor, and has the nuclear reprogramming activity
(C) A protein that consists of an amino acid sequence having 70% or more of sequence identity with the wild-type protein provided as an exemplary example of the nuclear reprogramming factor and has the nuclear reprogramming activity

In (B) described above, "mutation" may be any one of deletion, substitution, addition, and insertion or may be a combination thereof.

In (B) described above, "a plurality of amino acids" is not particularly limited as long as the resultant protein has the nuclear reprogramming activity. However, examples thereof include 2 to 30 amino acids, where 2 to 20 amino acids are preferable, 2 to 10 amino acids are more preferable, 2 to 5 amino acids are still more preferable, and 2 or 3 amino acids are particularly preferable.

In (C) described above, the sequence identity is not particularly limited as long as it is 70% or more. However, it is preferably 80% or more, more preferably 85% or more, and still more preferably 90% or more, 95% or more, 96% or more, 97% or more, 98% or more, or 99% or more.

In a case where the nuclear reprogramming factor is a protein, a protein transduction domain (PTD) may be linked for introduction into somatic cells.

In a case where the nuclear reprogramming factor is a gene or a gene product thereof, an aspect using only the gene may be good, an aspect using only the gene product may be good, or an aspect using both the gene and the gene product thereof may be good. In a case where the nuclear reprogramming factors are genes or gene products thereof, and two or more kinds of "genes or gene products thereof" are used in combination, an aspect in which a gene product is used for one gene and a gene is used for the other gene may be good.

It is preferable that the nuclear reprogramming factor is the gene or a combination of the genes exemplified above, where a form thereof is more preferably an expression vector.

### (Somatic cell)

The somatic cell is not particularly limited and can be appropriately selected depending on the intended purpose. Examples of the somatic cell include a somatic cell in the fetal stage and a mature somatic cell. Specific examples of the mature somatic cell include a tissue stem cell (a somatic stem cell) such as a mesenchymal stem cell, a hematopoietic stem cell, an adipose tissue-derived stromal cell, an adipose tissue-derived stromal stem cell, a neural stem cell, or a spermatogonial stem cell; a tissue progenitor cell; and a differentiated cell such as a fibroblast, an epithelial cell, a lymphocyte, or a muscle cell.

The biological species from which somatic cells are derived is not particularly limited and can be appropriately selected depending on the intended purpose. The biological species from which somatic cells are derived include any mammals such as a human, a mouse, a rat, a cow, a sheep, a horse, and a monkey. The biological species from which somatic cells are derived and the biological species from which nuclear reprogramming factors are derived may not be the same; however, they are preferably the same. The biological species from which the H1foo gene or H1foo protein which is contained in the quality improving agent for iPS cells according to the above embodiment is derived may not be the same as the biological species from which somatic cells are derived; however, they are preferably the same.

An individual from which somatic cells are derived is not particularly limited and can be appropriately selected depending on the intended purpose. However, in a case where iPS cells produced by the method according to the present embodiment are used for use application in regenerative medicine, an individual who is targeted for the regenerative medicine, or another individual of which the MHC type is the same as or substantially the same as the individual who is targeted for the regenerative medicine is preferable from the viewpoint of rejection reaction. Here, the above-described phrase that MHC type is substantially the same refers to that in a case where cells obtained by inducing differentiation from the above-described somatic cell-derived iPS cells are transplanted into an individual, the MHC type matches to the extent that the engraftment of the transplanted cells is possible by using an immunosuppressant or the like.

The somatic cell may be a recombinant somatic cell into which a heterologous gene has been introduced to facilitate the selection of iPS cells. Specific examples of the recombinant somatic cell include a recombinant somatic cell in which at least any one of a reporter gene and a drug resistance gene has been incorporated into a locus of a gene that is high expressed specifically in a pluripotent cell. Examples of the gene that is highly expressed specifically in a pluripotent cell include an Fbx15 gene, a Nanog gene, and an Oct3/4 gene. Examples of the reporter gene include a green fluorescent protein (GFP) gene, a luciferase gene, and a β-galactosidase gene. Examples of the drug resistance gene include a blasticidin gene, a hygromycin gene, a puromycin resistance gene, and a neomycin resistance gene.

The culture conditions for the somatic cell are not particularly limited and can be appropriately selected depending on the intended purpose. Examples thereof include culture conditions of a culture temperature of about 37°C, a CO₂ concentration of about 2% to 5%, and an O₂ concentration of about 5% to 20%. The culture medium that is used in the culture of the somatic cell is not particularly limited and can be appropriately selected depending on the intended purpose. Examples thereof include a minimum essential medium (MEM), a Dulbecco's Modified Medium (DMEM), an RPMI1640 medium, a 199 medium, and an F12 medium, which contain 5% by mass to 20% by mass of serum. In this specification, the simple notation of "%" for concentration means "% by volume".

### (Introduction into somatic cells)

A method of introducing the nuclear reprogramming factor and the quality improving agent for iPS cells (hereinafter, simply referred to as the "quality improving agent") according to the above embodiment into somatic cells is not particularly limited and can be appropriately selected depending on the intended purpose. Considering the ease of introduction into somatic cells, the nuclear reprogramming factor is preferably introduced into somatic cells in a form of a gene or an mRNA of the gene rather than in a form of a protein and more preferably introduced into somatic cells in a form of a gene. In particular, the nuclear reprogramming factor is preferably introduced into somatic cells in a form of an expression vector. Similarly, a polynucleotide contained in the quality improving agent is also preferably in a form of an expression vector.

A method of introducing an expression vector into somatic cells is not particularly limited and can be appropriately selected depending on the intended purpose. Examples of the method of introducing an expression vector into somatic cells include a lipofection method, a microinjection method, a DEAE dextran method, a gene gun method, an electroporation method, and a calcium phosphate method. In a case where the expression vector is a virus vector, examples of the method of infecting somatic cells with the virus vector include a polybrene method.

In a case where a Sendai virus vector is used as an expression vector, a Sendai virus vector carrying a nuclear reprogramming factor or a quality improving agent or carrying a nuclear reprogramming factor and a quality improving agent is introduced into somatic cells by adding the Sendai virus vector (the Sendai virus particles) to a culture medium containing somatic cells to infect the somatic cells. The dose of the Sendai virus vector can be adjusted appropriately; however, the infection can be carried out, for example, at a multiplicity of infection (MOI) of 0.1 or more, and preferably at a multiplicity of infection of 0.3 or more, 0.5 or more, 1 or more, 2 or more, or 3 or more, and 100 or less, and preferably 90 or less, 80 or less, 70 or less, 60 or less, 50 or less, 40 or less, 30 or less, 20 or less, 10 or less, or 5 or less. Infection is carried out preferably at an MOI of 0.3 to 100, and more preferably an MOI of 0.5 to 50, an MOI of 1 to 40, an MOI of 1 to 30, an MOI of 2 to 30, or an MOI of 3 to 30.

In a case where the Sendai virus vector is in a form of RNP, it can be introduced into cells by a method such as electroporation, lipofection, or microinjection.

In a case where the nuclear reprogramming factor is an mRNA, a method of introducing mRNA (messenger RNA) into somatic cells is not particularly limited, and a known method can be appropriately selected and used. For example, mRNA can be introduced into somatic cells using a commercially available RNA transfection reagent such as Lipofectamine (registered trade name) Messenger MAX (manufactured by Thermo Fisher Scientific, Inc. under the brand of Life Technologies)).

In a case where the nuclear reprogramming factor is a protein, a method of introducing a protein into somatic cells is not particularly limited, and a known method can be appropriately selected and used. Examples of such a method include a method using a protein introduction reagent, a method using a protein transduction domain (PTD) fusion protein, and a microinjection method. In a case where the quality improving agent contains a fusion protein of the H1foo protein and the destabilization domain, it can be also introduced into somatic cells by appropriately selecting a known method.

The following protein introduction reagents are commercially available: BioPOTER (registered trade name) Protein Delivery Reagent (manufactured by Gene Therapy Systems, Inc), and Pro-Ject^{™} Protein Transfection Reagent (manufactured by PIERCE), which are based on a cationic lipid; Profect-1 (manufactured by Targeting Systems) based on a lipid; Penetratin Peptide (manufactured by Q biogene) and Chariot Kit (manufactured by Active Motif) based on a membrane-permeable peptide; and GenomONE (manufactured by ISHIHARA SANGYO KAISHA, LTD.) using an HVJ envelope (an inactivated Sendai virus). Protein transduction can be carried out according to the protocol attached to these reagents; however, the general procedure is as follows. A protein is diluted in a suitable solvent (for example, a buffer solution such as PBS, HEPES, or the like), an introduction reagent is added thereto and incubated at room temperature for about 5 to 15 minutes to form a complex, and this complex is added to cells subjected to the culture medium replacement with a serum-free medium, and the cells are incubated at 37°C for 1 hour to several hours. Thereafter, the culture medium is removed and replaced with a serum-containing medium.

Examples of PTD include those mentioned in the section of "[Quality improving agent for iPS cells], <Second embodiment>, (Fusion protein)", which are described above. Also in a case where PTD is used, the procedure can be carried out in the same manner as described above except that the protein introduction reagent is not added.

The microinjection method is a method in which a protein solution is put into a glass needle having a tip diameter of about 1 µm and introduced into cells by puncturing, whereby a protein can be reliably introduced into cells. In the past, in a mouse and a human, a method in which iPS cells are established by introducing a nuclear reprogramming factor in a form of a protein together with a cell penetrating peptide (CPP) such as polyarginine or TAT has been developed, and these methods can be also used (Cell Stem Cell, 4:381-384 (2009)).

In the method of producing iPS cells according to the present embodiment, the number of times of the introduction of the nuclear reprogramming factor and the quality improving agent into somatic cells may be once or twice or more. The period of the introduction thereof is not particularly limited and can be appropriately selected depending on the intended purpose. All of the nuclear reprogramming factor and the quality improving agent may be introduced at the same time, or a part or all of them may be introduced at different times. All of the nuclear reprogramming factors and the quality improving agent are preferably introduced into somatic cells at the same time. The number of times of introduction is preferably once.

In a case where the nuclear reprogramming factor is a gene or a gene product thereof, the amount of the nuclear reprogramming factor to be introduced into somatic cells is not particularly limited as long as it is capable of reprogramming the nuclei of the somatic cell, and all genes or gene products to be used may be introduced in equal amounts or may be introduced in different amounts. As an example of a case of using a gene as a gene or a gene product thereof, a method of introducing the Oct3/4 gene to be a large amount (for example, about 3 times the amount) with respect to the Sox2 gene, the Klf4 gene, the c-Myc gene, or the L-Myc gene is mentioned (PNAS 106 (31): 12759-12764 (2009), J. Biol. Chem. 287 (43): 36273-36282 (2012)).

In a case where the nuclear reprogramming factor that is used in the method of producing iPS cells according to the present embodiment is a low-molecular-weight compound, the contact of somatic cells with such a low-molecular-weight compound can be carried out by dissolving the low-molecular-weight compound in an aqueous or nonaqueous solvent at an appropriate concentration, adding the prepared low-molecular-weight compound solution in a culture medium suitable for culturing somatic cells (for example, a minimum essential medium (MEM), a Dulbecco's Modified Eagle Medium (DMEM), an RPMI1640 medium, a 199 medium, and an F12 medium, which contains about 5% to 20% of fetal bovine serum) so that the concentration of the low-molecular-weight compound is sufficient for the nuclear reprogramming to occur in somatic cells and no cytotoxicity is observed, and culturing the cells for a certain period of time. The concentration of the low-molecular-weight compound, which is a nuclear reprogramming factor, varies depending on the kind of the low-molecular-weight compound to be used; however, it can be appropriately selected within a range of about 0.1 nM to about 100 nM. The contact period is not particularly limited as long as it is sufficient for the nuclear reprogramming of cells to be achieved; however, it generally sufficient to allow the low-molecular-weight compound to be present together in the culture medium until positive colonies appear.

### <<Another step>>

The method of producing iPS cells according to the present embodiment can further include another step in addition to the introduction step, as necessary. The other step is not particularly limited as long as the effect of the present invention is not impaired, and it can be appropriately selected depending on the intended purpose. Examples thereof include a step of culturing somatic cells (hereinafter, also simply referred to as "post-introduction cells") into which a nuclear reprogramming factor and a quality improving agent have been introduced (hereinafter, also simply referred to as a "post-introduction cell culture step").

### (Post-introduction cell culture step)

The post-introduction cell culture step is a step of culturing post-introduction cells. The culture conditions for the post-introduction cells are not particularly limited, and the generally used culture conditions for stem cells can be used, where examples thereof include the culture conditions suitable for ES cell culture. Examples of such conditions include culture conditions of a culture temperature of about 37°C, a CO₂ concentration of about 2% to 5%, and an O₂ concentration of about 5% to 20%. The culture medium that is used in the culture of the post-introduction cells is not particularly limited and can be appropriately selected depending on the intended purpose. In a case of mouse cells, culture is carried out by adding Leukemia Inhibitory Factor (LIF) as a differentiation inhibitory factor to a normal culture medium. On the other hand, in a case of human cells, LIF, or basic fibroblast growth factor (bFGF) and/or stem cell factor (SCF) is added depending on the culture conditions. In general, cells are cultured in the coexistence of a mouse embryonic fibroblast (MEF) that has been treated with radiation or antibiotics to stop cell division, which serves as feeder cells. An STO cell or the like is generally used as MEF; however, an SNL cell (McMahon, A. P. & Bradley, A. Cell 62, 1073-1085 (1990)) or the like is often used for inducing iPS cells. Co-culturing with feeder cells may be started before the introduction of the nuclear reprogramming factor and the quality improving agent or may be started at the time of the introduction or after the introduction (for example, 1 to 10 days later after the introduction).

The period of the post-introduction cell culture step is not particularly limited, and it can be appropriately selected depending on the intended purpose.

According to the method of producing iPS cells according to the present embodiment, after the nuclear reprogramming factor and the quality improving agent are introduced into the somatic cell, it is possible to regulate at least one of the period of the existence and the amount of the H1foo protein in cells by the regulatory sequence contained in the quality improving agent. Therefore, the period of the existence or the amount of the H1foo protein can be suitably regulated, and as a result, iPS cells having high quality can be obtained.

### <Second embodiment>

In one embodiment, the present invention provides a method of producing iPS cells, including a step of introducing a nuclear reprogramming factor and an H1foo gene into somatic cells. The H1foo protein expressed from the H1foo gene introduced into the somatic cells is regulated in at least one of the period of the existence and the amount in the somatic cells.

### <<Introduction step>>

The production method according to the present embodiment includes a step of introducing a nuclear reprogramming factor and an H1foo gene into somatic cells.

### (Nuclear reprogramming factor)

The nuclear reprogramming factor is the same as that described in "<First embodiment>" described above. Examples of the preferred nuclear reprogramming factor also include the same ones as those mentioned in "<First embodiment>" described above.

### (H1foo gene)

The H1foo gene is the same as those described in "[Quality improving agent for iPS cells], <First embodiment>, and (Hlfoo gene)", which are described above. Preferred examples of the H1foo gene also include the same ones as those mentioned in "[Quality improving agent for iPS cells], <First embodiment>, and (H1foo gene)", which are described above.

In the method of producing iPS cells according to the present embodiment, the H1foo protein expressed from the H1foo gene introduced into somatic cells is regulated in at least one of the period of the existence and the amount in somatic cells. The method of regulating the period of the existence and the amount of the H1foo protein in somatic cells is not particularly limited, and a known method can be used.

Preferred examples of the method of regulating the period of the existence and the amount of the H1foo protein preferably include a method using the regulatory sequence mentioned in "[Quality improving agent for iPS cells], <First embodiment>, and (Regulatory sequence)". Examples thereof include a method in which a destabilization domain gene is linked to the H1foo gene and then expressed as a fusion protein of the H1foo protein and the destabilization domain in a case of introduced into somatic cells. In this case, due to having the destabilization domain, the fusion protein is rapidly degraded by the proteasome after being produced in the cells. The time taken until a fusion protein is generated by transcription and translation from a fusion gene of the H1foo gene and the destabilization domain gene and degraded after the generation by the proteasome is, for example, within 6 hours, preferably within 4 hours, more preferably within 3 hours, and particularly preferably within 2 hours.

The period of the existence and the amount of the fusion protein may be regulated by the addition of a stabilizing substance. For example, in a case of adding a stabilizing substance to a culture medium of the post-introduction cells, or the cells are recovered from the culture medium and transferred to a culture medium containing no stabilizing substance, the fusion protein is allowed to be present in the cells at a desiredperiod . In addition, the intracellular amount of the fusion protein can be regulated by adjusting the amount of the stabilizing substance added.

Alternatively, a stimulus-responsive promoter may be linked upstream of the H1foo gene so that the H1foo gene is expressed under the regulation of the stimulus-responsive promoter in a case of being introduced into somatic cells. In this case, the period of the existence and the amount of the H1foo protein can be regulated by applying a "stimulus" to which a stimulus-responsive promoter responds, to the post-introduction cells. In a case where the stimulus-responsive promoter is a tetracycline-responsive promoter, it is possible to use tetracycline or a derivative thereof (doxycycline or the like) to regulate the expression timing and expression level of the H1foo gene, and as a result, it is possible to regulate the period of the existence and the amount of the H1foo protein in the post-introduction cells.

### (Introduction into somatic cells)

Examples of the method of introducing the nuclear reprogramming factor and the H1foo gene into somatic cells include the same methods as the methods mentioned in "<First embodiment>" described above.

### <<Another step>>

The method of producing iPS cells according to the present embodiment can further include another step in addition to the introduction step, as necessary. The other step is not particularly limited as long as the effect of the present invention is not impaired, and it can be appropriately selected depending on the intended purpose. Examples of the other step include the post-introduction cell culture step, as in the case of "<First embodiment>" described above. The post-introduction cell culture step can be carried out in the same manner as in "<First embodiment>" described above.

The method of producing iPS cells according to the present embodiment may include a step of regulating at least one of the amount and the period of the existence of the H1foo protein in the post-introduction cells (hereinafter, referred to as an "H1foo protein regulation step"). The regulation of the amount and the period of the existence of the H1foo protein can be carried out using a stabilizing substance, for example, in a case where the H1foo protein is expressed as a fusion protein fused with a destabilization domain. In addition, for example, in a case where the H1foo gene is expressed under the regulation of a stimulus-responsive promoter, the amount and the period of the existence of the H1foo protein can be regulated by applying a stimulus to which a responsive promoter responds, to the post-introduction cells.

The amount and the period of the existence of the H1foo protein in the post-introduction cells are regulated so that in a case where iPS cells are produced, the expression level of the H1foo protein is 50% or less, preferably 30% or less, and more preferably 20% or less of the maximum expression level until the reprogramming of somatic cells is completed. More specifically, the H1foo protein introduced into cells is regulated so that the expression level thereof in the cells is 50% or less, preferably 30% or less, and more preferably 20% or less 24 hours after the expression in the cells has reached the maximum. Alternatively, the duration period of the expression of the H1foo gene after the introduction of the nuclear reprogramming factor into somatic cells may be within 24 hours after the introduction. Alternatively, the amount of the H1foo protein in cells into which the nuclear reprogramming factor has been introduced is less than about 50% of the maximum amount of the H1foo protein after the introduction at about 5 days after the introduction.

According to the method of producing iPS cells according to the present embodiment, it is possible to suitably regulate at least one of the period of the existence and the amount of the H1foo protein in cells. As a result, iPS cells having high quality can be obtained.

### [iPS cell]

In one embodiment, the present invention provides iPS cells produced by the method of producing iPS cells according to the above embodiment.

The iPS cell is a pluripotent stem cell induced from a somatic cell and has pluripotency and self-replication ability. The pluripotency means an ability to differentiate into all three germ layer lineages. In addition, the self-replication ability means an ability to proliferate while maintaining the undifferentiated state.

A known method can be used to check whether or not cells produced by the method of producing iPS cells according to the above embodiment are iPS cells. For example, it is possible to check whether cells are iPS cells by the expression of undifferentiation markers, the embryoid body forming ability, and/or the ability to form chimeras.

The cells produced by the method of producing iPS cells according to the above embodiment may be iPS cells containing a nuclear reprogramming factor and the quality improving agent for iPS cells according to the above embodiment. The cells produced by the method of producing iPS cells according to the above embodiment may be cells containing a nuclear reprogramming factor, an H1foo gene, and a regulatory sequence. The cells produced by the method of producing iPS cells according to the above embodiments may be cells containing a nuclear reprogramming factor and a fusion gene of the H1foo gene and the regulatory sequence (for example, the destabilization domain gene). The cells produced by the method of producing iPS cells according to the above embodiment may be cells containing a nuclear reprogramming factor and an H1foo gene functionally linked to a stimulus-responsive promoter.

The iPS cells produced by the production method according to the above embodiment are iPS cells having high quality as compared with the iPS cells produced by the methods in the related art, since the period of the existence and the amount of the H1foo protein are suitably regulated in the post-introduction cells. In particular, in the iPS cells according to the present embodiment, the number of formed colonies in which Tra-1-60 is expressed is increased. In addition, the ability to generate naive-type iPS cells is improved. The fact that a cell is a naive-type iPS cell can be confirmed by the facts that colonies have a naive type-specific dome shape and that a naive type-specific gene is highly expressed. In addition, the ability to differentiate into a target cell (for example, a cardiomyocyte or a primitive endoderm cell) is improved. Further, a cell population of the iPS cells produced by the production method according to the above embodiment can be a uniform cell population having no variation in gene expression.

### [Composition for producing iPS cells]

In one embodiment, the present invention provides a composition for producing iPS cells. The composition for producing iPS cells according to the present embodiment contains a nuclear reprogramming factor and the quality improving agent for iPS cells according to the above embodiment.

The nuclear reprogramming factor is the same as those described in "[Method of producing iPS cells] and <First embodiment>" described above. Examples of the preferred nuclear reprogramming factor also include the same ones as those mentioned in "<First embodiment>" described above.

The quality improving agent for iPS cells is the same as that described in "[Quality improving agent for iPS cells]" described above. Examples of the preferred quality improving agent also include the same ones as those mentioned in "[Quality improving agent for iPS cells]" described above.

In the composition for producing iPS cells according to the present embodiment, the amounts of each nuclear reprogramming factor and quality improving agent are not particularly limited, all of which may be the same amount or may be amounts different from each other. For example, in a case where the Oct3/4 gene, the Sox2 gene, the Klf4 gene, the c-Myc gene, or the L-Myc gene is used as a nuclear reprogramming factor, the amount of the Oct3/4 gene may be such that it is a large amount, for example, about 3 times the amount of the Sox2 gene, the Klf4 gene, or the c-Myc gene, or L-Myc gene.

The composition for producing iPS cells according to the present embodiment may contain other components in addition to the nuclear reprogramming factor and the quality improving agent. Examples of the other components, which are not particularly limited, include a buffer, a gene introduction reagent, and a protein introduction reagent.

### [Third embodiment]

The present invention provides, as another embodiment, a method of improving the quality of iPS cells, including a step of introducing the quality improving agent for iPS cells according to the above embodiment into somatic cells.

The present invention provides, as another embodiment, use of a fusion gene of an H1foo gene and a destabilization domain gene in the production of a quality improving agent for iPS cells.

The present invention provides, as another embodiment, use of a fusion protein of an H1foo protein and a destabilization domain in the production of a quality improving agent for iPS cells.

The present invention provides, as another embodiment, use of an H1foo gene linked downstream of a stimulus-responsive promoter in the production of a quality improving agent for iPS cells.

The present invention provides, as another embodiment, use of a nuclear reprogramming factor and a fusion gene of an H1foo gene and a destabilization domain in the production of a composition for producing iPS cells.

The present invention provides, as another embodiment, use of a nuclear reprogramming factor and a fusion protein of an H1foo protein and a destabilization domain in the production of a composition for producing iPS cells.

The present invention provides, as another embodiment, use of a nuclear reprogramming factor and an H1foo gene linked downstream of a stimulus-responsive promoter in the production of a composition for producing iPS cells.

The present invention provides, as another embodiment, a kit for producing iPS cells, containing a nuclear reprogramming factor and the quality improving agent for iPS cells according to the above embodiment. The nuclear reprogramming factor and the quality improving agent may be divided into separate containers or may be combined in one container, or any number of them may be put together in containers. In addition, a gene introduction reagent, a protein introduction reagent, a packaging cell, a buffer, a dilution solution, or the like may be contained.

### [Examples]

Hereinafter, the present invention will be described in detail according to Examples; however, the present invention is not limited to these Examples. All experiments below are carried out in accordance with the guideline for animal experiments and DNA experiments of Keio University and Kyoto University, is approved by the Ethics Committee of Keio University and Kyoto University, and comply with the Guide for the Care and Use of Laboratory Animals of National Institutes of Health.

### [Example 1] Construction of Sendai virus vector containing H1FOO gene

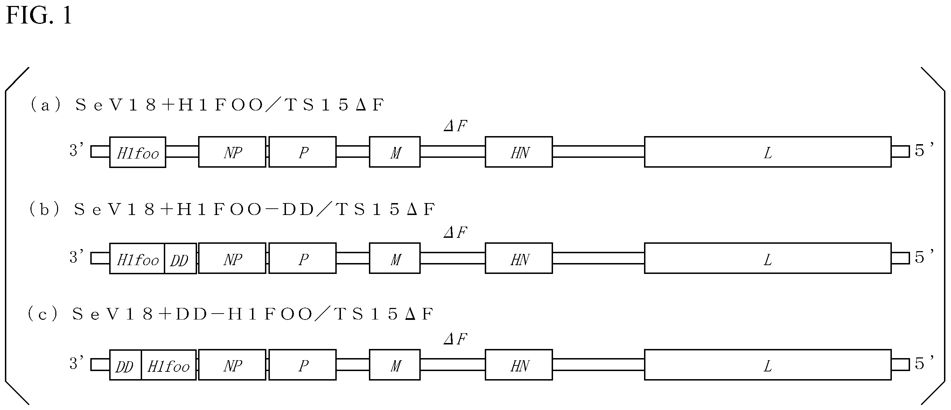

Using a Sendai virus vector (SeV18; manufactured by ID Pharma Co., Ltd.) and ProteoTuner^{™} Shield System (manufactured by Clontech), three Sendai virus vectors containing an H1FOO cDNA (Teranishi, T., et al. Rapid replacement of somatic linker histones with the oocyte-specific linker histone H1foo in nuclear transfer. Developmental Biology 266, 76-86 (2004).) were constructed (FIG. 1).
(a) SeV18+H1FOO/TS15ΔF is a vector that does not contain a destabilization domain (DD) of the ProteoTuner^{™} Shield System.
(b) SeV18+H1FOO-DD/TS15ΔF is a vector containing a sequence in which a DD coding sequence is added to the 3' terminal of the H1FOO cDNA. The vector of (b) expresses a fusion protein in which DD is added to the C-terminal of H1FOO to promote the degradation of H1FOO.
(c) SeV18+DD-H1FOO/TS15ΔF is a vector containing a sequence in which a DD coding sequence is added to the 5' terminal of the H1FOO cDNA. The vector of (c) expresses a fusion protein in which DD is added to the N-terminal of H1FOO to promote the degradation of H1FOO.

Each of the sequences in the vectors of (a) to (c) described above is shown in the following sequence identification numbers.
DD coding sequence: SEQ ID NO: 13
H1FOO-DD coding sequence: SEQ ID NO: 15
DD-H1FOO coding sequence: SEQ ID NO: 17

In addition, Table 1 shows primer sequences that are used to construct the above vectors of (a) to (c).

**[Table 1]**

| | Primer name | Sequence | SEQ ID NO |
|---|---|---|---|
| Preparation of H1FOO fragment | Not1_H1FOO_N | TAAGCGGCCGCCAAGGTTCACTTATGGCCCCCGCGACG | 19 |
| | H1FOO_EIS_Not1_C | TTAGCGGCCGCGATGAACTTTCACCCTAAGTTTTTCTTACTA CGGCTAAGCTTCAGCCCTCTGGCTG | 20 |
| Preparation of H1FOO-DD fragment | Not_H1FOO_N | ATATGCGGCCGCGACGCCACCATGGCCCCCGCGACGGCTCC CAG | 21 |
| | H1FOO_DD_C | CCACCTGCACTCCAGCTTCAGCCCTCTGGC | 22 |
| | H1FOO DD N | AGGGCTGAAGCTGGAGTGCAGGTGGAAACC | 23 |
| | DD_EIS_Not1_C | ATATGCGGCCGCGATGAACTTTCACCCTAAGTTTTTCTTACT ACGGCTATTCCAGTTCTAGAAGCTCCAC | 24 |
| Preparation of DD-H1FOO fragment | Not1_DD_N (kozak-) | ATATGCGGCCGCCAAGGTTCAATGGGAGTGCAGGTGGAAA CC | 25 |
| | DD_H1FOO_C | GCCGTCGCGGGGGCTTCCAGTTCTAGAAG | 26 |
| | DD H1FOO N | CTTCTAGAACTGGAAGCCCCCGCGACGGC | 27 |

### [Example 2] Western blotting of H1FOO protein

Each of Sendai virus vectors of (a) to (c) described above, containing the H1FOO gene was introduced into a human skin fibroblast (a TIG120 strain, provided from Tokyo Metropolitan Institute of Gerontology, Kondo et al., Exp Cell Res. 1995 Oct; 220 (2): 501-4) at a multiplicity of infection (MOI) of 3, each cells were recovered after culturing for 5 days, and Western blotting was carried out using an anti-H1FOO antibody (HPA037992; manufactured by Sigma-Aldrich Co., LLC).

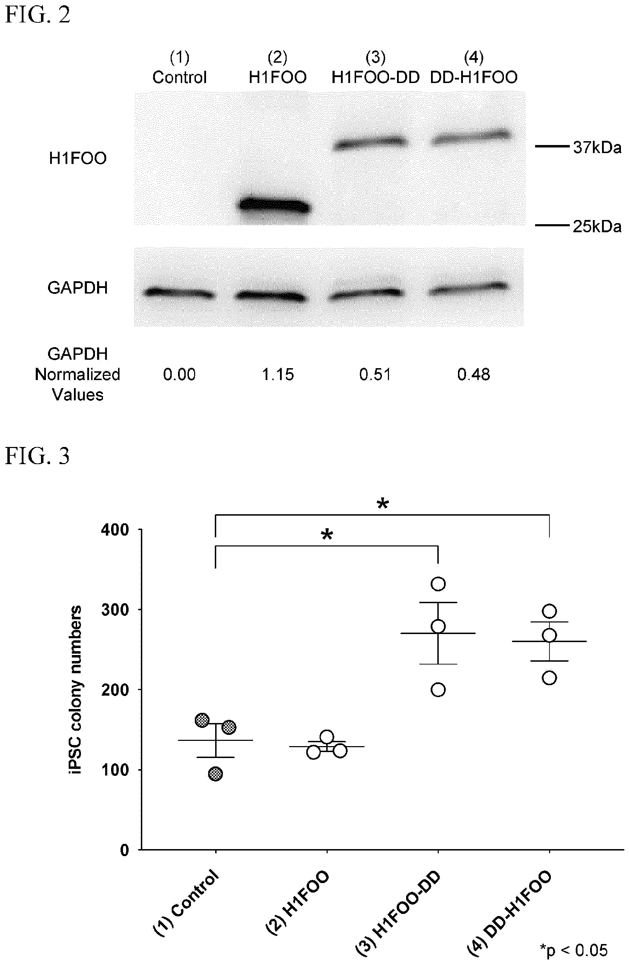

The results are shown in FIG. 2. In FIG. 2, (1) to (4) each shows a result obtained from human skin fibroblasts which are introduced with each vector of (1) Control not containing H1FOO, (2) H1FOO (vector (a)), (3) H1FOO-DD (vector (b)), and (4) DD-H1FOO (vector (c)). After 5 days, (2) H1FOO showed a significantly higher expression level of the H1FOO protein. On the other hand, in (3) H1FOO-DD and (4) DD-H1FOO, the expression level of the H1FOO-DD fusion protein or the DD-H1FOO fusion protein was decreased.

As (1) Control not containing H1FOO, a Sendai virus vector containing an Azami-Green gene in the vector of (a) instead of the H1FOO gene was used.

### [Example 3] Preparation and culture conditions for prime-type human iPS cells

The preparation of human iPS cells was carried out according to the protocol described in the following documents: Ban, H., Nishishita, N., Fusaki, N., Tabata, T., Saeki, K., Shikamura, M., Takada, N., Inoue, M., Hasegawa, M., Kawamata, S., Nishikawa, S., Efficient generation of transgene-free human induced pluripotent stem cells (iPSCs) by temperature-sensitive Sendai virus vectors, Proc Natl Acad Sci U S A 108, 14234-9, 2011; and Seki, T., Yuasa, S., Fukuda, K., Generation of induced pluripotent stem cells from a small amount of human peripheral blood using a combination of activated T cells and Sendai virus, Nature Protocols 7, 718-728, 2012. Using any one of the Sendai virus vectors of (a) to (c) prepared in Example 1 described above together with one or moreSendai virus vectors containing an Oct3/4 gene, a Sox2 gene, a Klf4 gene, and an L-Myc gene, prime-type iPS cells were prepared from human skin fibroblasts (a TIG120 strain) and peripheral blood mononuclear cells (product number: CTL-UP1, Lot: HHU20140120, Cellular Technology Ltd.) ( each of the cells wasinfected at a multiplicity of infection of 0.1 to 0.3). As a control, iPS cells (Control-iPS) were prepared using a Sendai virus vector containing an Azami-Green gene instead of the H1FOO gene in the vector of (a) described above. It is noted that this experiment was carried out according to the guideline for gene recombination experiments of Keio University and Kyoto University.

Human iPS cells prepared as described above were cultured and maintained in a StemFit AK02N (manufactured by Ajinomoto Co., Inc.) culture solution (hereinafter, referred to as a "prime-type human iPS cell culture solution") on a culture dish coated with iMatrix-511 (manufactured by Nippi. Inc.). The culture solution of the prime-type human iPS cells was replaced every 2 days, and the cells were passaged every 5 to 7 days using StemPro Accutase (manufactured by Thermo Fisher Scientific, Inc. under the brand of Gibco).

### [Example 4] Preparation and culture conditions for naive-type human iPS cells

The production of human iPS cells was carried out according to the protocol described in the following documents: Ban, H. et al., Proc Natl Acad Sci U S A 108, 14234-9, 2011; Seki, T. et al., Nature Protocols 7, 718-728, 2012; and Liu, X et al., Nature Methods 14, 1055-1062, 2017. Using any one of the Sendai virus vectors of (a) to (c) prepared in Example 1 described above together with one or more Sendai virus vectors containing an Oct3/4 gene, a Sox2 gene, a Klf4 gene, and an L-Myc gene, naive-type iPS cells were prepared from human skin fibroblast and human peripheral blood mononuclear cells (each of the cells wasinfected at a multiplicity of infection of 0.1-0.3). The fact that the prepared cells are naive-type iPS cells was confirmed from the facts that colonies have a naive type-specific dome shape and that a naive type-specific gene is highly expressed (in cases of genes of KLF17, TFCP2L1, PRDM14, and DPPA3, the confirmation was carried out by quantitative PCR and immunostaining). As a control, iPS cells (Control-iPS) were prepared using a Sendai virus vector containing an Azami-Green gene instead of the H1FOO gene in the vector of (a) described above. This experiment was carried out according to the guideline for gene recombination experiments of Keio University and Kyoto University.

The human iPS cells prepared as described above were cultured and maintained on a culture dish in which mouse embryonic fibroblasts subjected to radiation irradiation were seeded, in a culture solution (hereinafter, referred to as a "naive-type human iPS cell culture solution") obtained by adding CHIR99021 (manufactured by Sigma-Aldrich Co., LLC), PD0325901 (manufactured by Sigma-Aldrich Co., LLC), Go6983 (manufactured by Sigma-Aldrich Co., LLC), and Human recombinant leukemia inhibitory factor (manufactured by FUJIFILM Wako Pure Chemical Corporation) to NDiff227 (manufactured by Takara Bio Inc.). The culture solution of the naive-type human iPS cells was replaced every 2 days, and the cells were passaged every 3 to 4 days using StemPro Accutase (manufactured by Thermo Fisher Scientific, Inc. under the brand of Gibco). Using an incubator that enables the adjustment of the oxygen concentration, culture and passage were carried out under a hypoxic environment of an oxygen concentration of 5%.

### [Example 5] Comparison of iPS cell establishment efficiency by counting number of ALP-positive iPS cell colonies

According to the method described in Example 3 described above, (1) Control-iPS, (2) H1FOO-iPS, (3) H1FOO-DD-iPS, and (4) DD-H1FOO-iPS, which belong to the prime type, were prepared from human skin fibroblasts and subsequently cultured in the prime-type human iPS cell culture solution for 15 days, and the number of ALP-positive colonies was measured. Here, ALP is known as a stem cell marker.

The results are shown in FIG. 3. It was shown that in (3) H1FOO-DD-iPS and (4) DD-H1FOO-iPS, the number of colonies of the ALP-positive cell significantly increases as compared with (1) Control-iPS.

Next, in order to verify the difference in the effect depending on the original cell kind, (1) Control-iPS cells and (3) H1FOO-DD-iPS, which belong to the prime type, were prepared from human peripheral blood mononuclear cells and cultured in the prime-type human iPS cell culture solution for 15 days, and the number of ALP-positive colonies was measured. Further, in order to compare the efficiency of the naive-type iPS cell establishment, each of (1) Control-iPS cells and (3) H1FOO-DD-iPS cells, which belong to the naive type, was prepared from human skin fibroblasts and human peripheral blood mononuclear cells according to the method described in Example 4 described above and cultured in the naive-type human iPS cell culture solution for 15 days, and the number of ALP-positive colonies was measured.

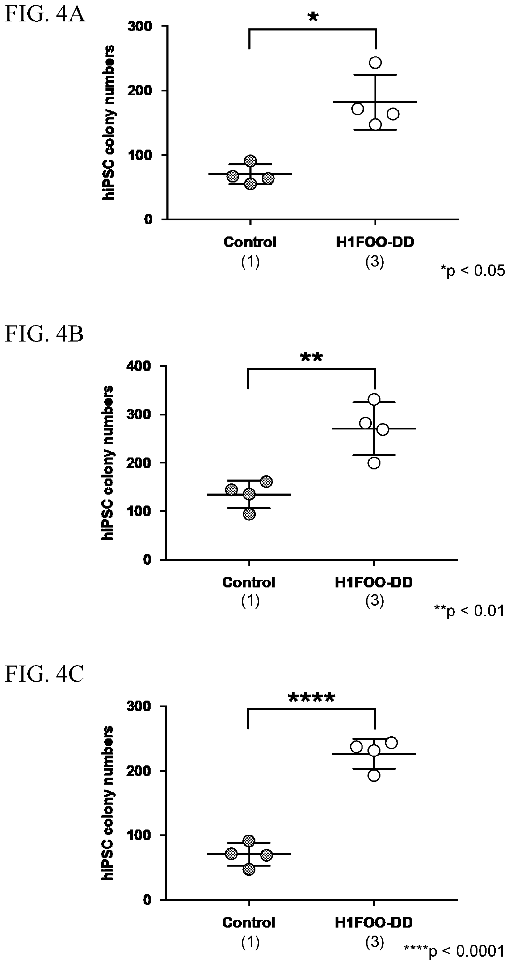

The results are shown in FIGS. 4A to 4C. In any one of Figure 4A (the prime-type human iPS cells established from human peripheral blood mononuclear cells), FIG. 4B (the naive-type human iPS cells established from human skin fibroblasts), and FIG. 4C (the naive-type human iPS cells established from human peripheral blood mononuclear cells), it was shown that the number of ALP-positive cell colonies significantly increases in (3) H1FOO-DD-iPS as compared with (1) Control-iPS, which is similar to the results in FIG. 3.

The above results showed that H1FOO-DD improves the efficiency of the human iPS cell establishment regardless of the original cell kind and the kind of iPS cells to be established.

### [Example 6] Comparison of variation in gene expression level

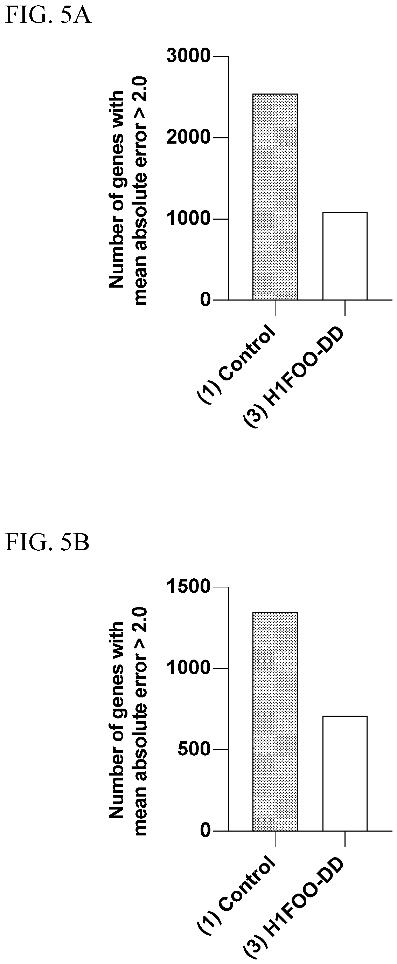

According to the method described in Example 3 described above, (1) Control-iPS and (3) H1FOO-DD-iPS, which belong to the prime type, were prepared from human skin fibroblasts. The culture was carried out for about 210 days (28 passages) in the prime-type human iPS cell culture solution, and then 8 clones were selected for each of (1) and (3) and subjected to RNA-seq. Based on the results of the RNA-seq data analysis, the number of genes having a mean absolute error (MAE) of the gene expression level of more than 2.0 (having a large variation) was obtained in the 8 clones of (1). By the same method, the number of genes having an MAE of more than 2.0 was also obtained in the 8 clones of (3). The results are shown in FIG. 5A.

According to the method described in Example 4 described above, (1) Control-iPS and (3) H1FOO-DD-iPS, which belong to the naive type, were prepared from human skin fibroblasts. The culture was carried out for about 190 days (45 passages) in the naive-type human iPS cell culture solution, and then 8 clones were selected for each of (1) and (3) and subjected to RNA-seq. By the same method as in the prime-type human iPS cells, the number of genes having an MAE of more than 2.0 was obtained in the 8 clones of each of (1) and (3). The results are shown in FIG. 5B.

In both the prime-type and naive-type human iPS cells, the number of genes having a large variation in expression level between clones was suppressed to about half in (3) H1FOO-DD-iPS as compared with (1) Control-iPS. These results show that the global gene expression is more uniform between clones in H1FOO-DD-iPS.

### [Example 7] Comparison of variation in DNA methylation

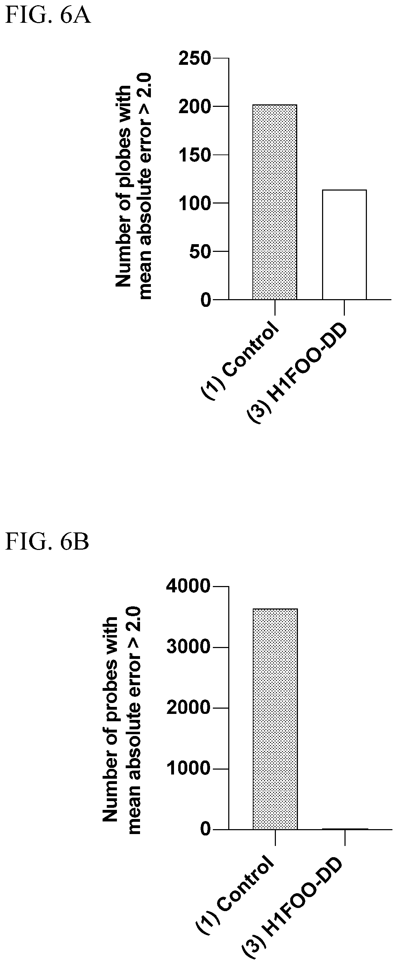

For 8 clones of (1) Control-iPS and 8 clones of (3) H1FOO-DD-iPS, which are the prime-type human iPS cells selected in Example 6 described above, analysis of DNA methylation was carried out using a DNA methylation array (Infinium Human Methylation 450K BeadChip Kit, Illumina). Based on the results of the DNA methylation array analysis, the number of methylation probes having a mean absolute error (MAE) of more than 2.0 (having a large variation) was obtained in the 8 clones of (1). By the same method, the number of genes having an MAE of more than 2.0 was also obtained in the 8 clones of (3). The results are shown in FIG. 6A.

For 8 clones of (1) Control-iPS and 8 clones of (3) H1FOO-DD-iPS, which are the naive-type human iPS cells selected in Example 6 described above, analysis of DNA methylation was carried out using a DNA methylation array. By the same method as in the prime-type human iPS cells, the number of methylation probes having an MAE of more than 2.0 was obtained in the 8 clones of each of (1) and (3). The results are shown in FIG. 6B.

In both the prime-type and naive-type human iPS cells, the number of methylation probes having a large variation between clones was suppressed to about half in (3) H1FOO-DD-iPS as compared with (1) Control-iPS. These results show that the DNA methylation is more uniform between clones in H1FOO-DD-iPS.

### [Example 8] Comparison of cardiomyocyte differentiation potency

According to the method described in Example 3 described above, (1) Control-iPS and (3) H1FOO-DD-iPS, which belong to the prime type, were prepared from human peripheral blood mononuclear cells. Three clones were selected for each of (1) and (3) and cultured in a myocardial differentiation induction medium to carry out induction of differentiation into cardiomyocytes (Tohyama, S., et al. (2013). Cell Stem Cell 12 (1): 127-137.). Cells were harvested on the 10th day after the start of culture in the myocardial differentiation induction medium. The expression of TNNT2 in the recovered cells was detected using a flow cytometer, and the proportion of TNN2-positive cells was calculated. Here, TNNT2 is a representative cardiomyocyte marker. Each clone was subjected to a test for differentiation induction into cardiomyocytes six times.

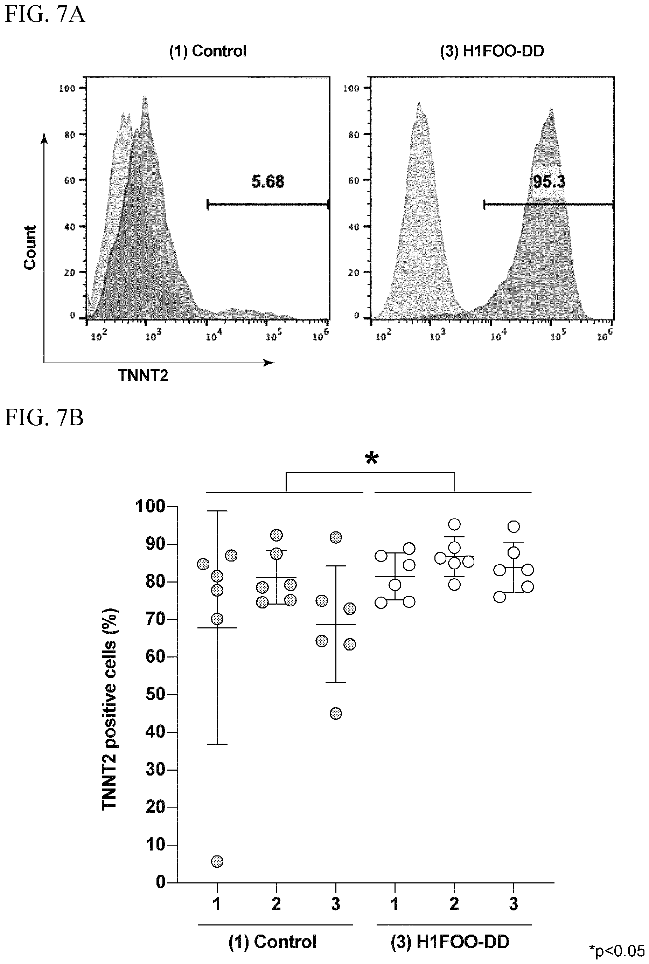

FIG. 7A shows measurement examples obtained by measuring the proportion (%) of TNNT2-positive cells by a flow cytometer. The left figure is a measurement example of (1) Control-iPS, and the right figure is a measurement example of (3) H1FOO-DD-iPS. In (1) Control-iPS, only about 6% of all cells were TNNT2-positive, and thus the induction of differentiation into cardiomyocytes was poor. In (3) H1FOO-DD-iPS, about 95% of all cells were strongly TNNT2-positive, and thus good myocardial differentiation potency was exhibited.

FIG. 7B shows the proportion of TNNT2-positive cells in each clone. In (3) H1FOO-DD-iPS, the proportion of TNNT2-positive cells was high as compared with (1) Control-iPS. In addition, it was confirmed that the variation between the tests for differentiation induction into cardiomyocytes is small in (3) H1FOO-DD-iPS as compared with (1) Control-iPS, and thus it is possible to more stably induce differentiation into cardiomyocytes. These results show that H1FOO-DD-iPS has excellent cardiomyocyte differentiation potency and can differentiate into cardiomyocytes having higher uniformity.

### [Example 9] Comparison of endoderm differentiation potency

According to the method described in Example 3 described above, (1) Control-iPS and (3) H1FOO-DD-iPS, which belong to the prime type, were prepared from human skin fibroblasts. Eight clones were selected for each of (1) and (3) and induced to differentiate into three germ layer components using a STEMdiff Trilineage differentiation kit (Cat: ST-05230, VERITAS). The cells were harvested on the 5th day after the start of differentiation induction. In the recovered cells, the expression of endoderm-related markers was semi-exhaustively evaluated by qPCR using a TaqMan hPSC Scorecard kit (Cat: A15876, ThermoFisher SCIENTIFIC, Inc.).

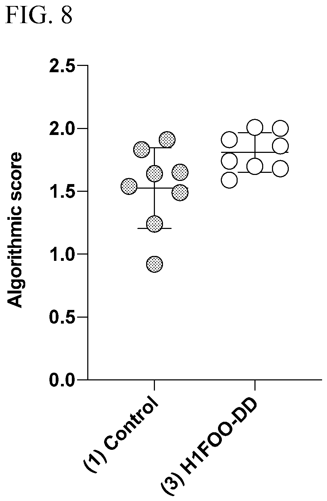

The results are shown in FIG. 8. In (3) H1FOO-DD-iPS, the algorithmic score was high as compared with (1) Control-iPS. In addition, it was confirmed that the variation between the clones is small in (3) H1FOO-DD-iPS as compared with (1) Control-iPS, and thus it is possible to more stably induce differentiation into the endoderm. These results show that H1FOO-DD-iPS has excellent endoderm differentiation potency and can differentiate into the endoderm having higher uniformity.

### [Example 10] Comparison of hepatocyte differentiation potency (1)

According to the method described in Example 3 described above, (1) Control-iPS and (3) H1FOO-DD-iPS, which belong to the prime type, were prepared from human peripheral blood mononuclear cells. Ten clones were selected for each of (1) and (3) and induced to differentiate into hepatocytes (one kind of terminally differentiated endodermal cells) (Kajiwara, M., et al. (2012). Proc Natl Acad Sci U S A 109(31): 12538-12543.; Takebe, T., et al. (2017). Cell Rep 21(10): 2661-2670.). The cells were harvested on the 21st day after the start of differentiation induction. In the recovered cells, ASGR1, which is one of the representative mature hepatocyte markers, was quantitatively evaluated by qRT-PCR.

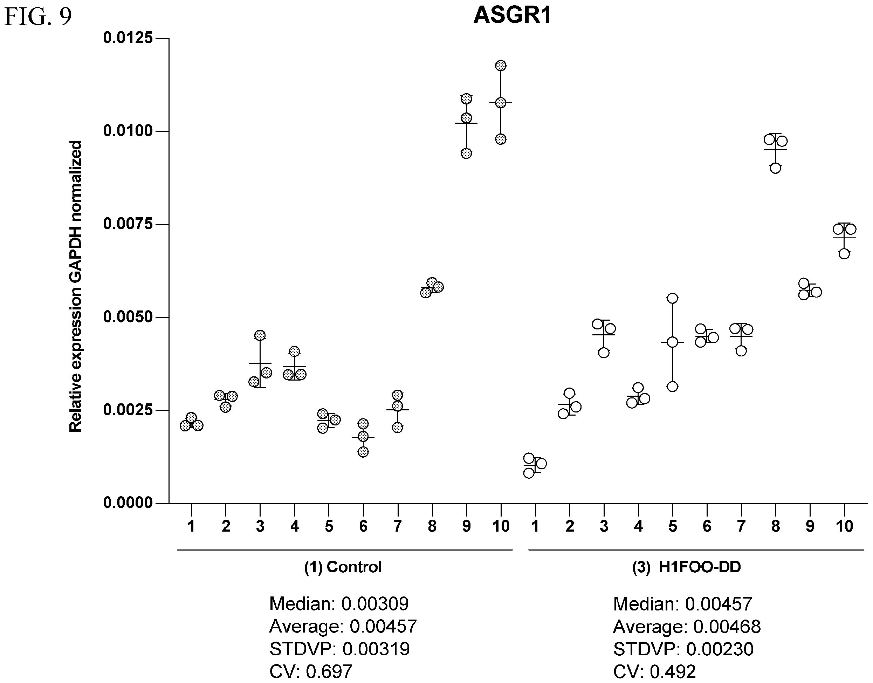

The results are shown in FIG. 9. In (3) H1FOO-DD-iPS, the expression level of AGSR1 was high on the whole as compared with (1) Control-iPS, and the median value and the average value were high. In addition, it was confirmed that the variation (STDVP, CV) between the clones is small in (3) H1FOO-DD-iPS as compared with (1) Control-iPS, and thus it is possible to more stably induce differentiation into hepatocytes. These results show that H1FOO-DD-iPS has excellent hepatocyte differentiation potency and can differentiate into hepatocytes having higher uniformity.

### [Example 11] Comparison of hepatocyte differentiation potency (2)

According to the method described in Example 3 described above, (1) Control-iPS and (3) H1FOO-DD-iPS, which belong to the prime type, were prepared from human peripheral blood mononuclear cells. Ten clones were selected for each of (1) and (3) and induced to differentiate into hepatocytes in the same manner as in Example 11. The supernatant was harvested on the 21st day after the start of differentiation induction. The amount of secreted Albumin in the recovered culture supernatant was quantitatively evaluated by ELISA. It is noted that mature hepatocytes produce and secrete Albumin, and thus Albumin, like ASGR1, is used as a marker for mature hepatocytes.

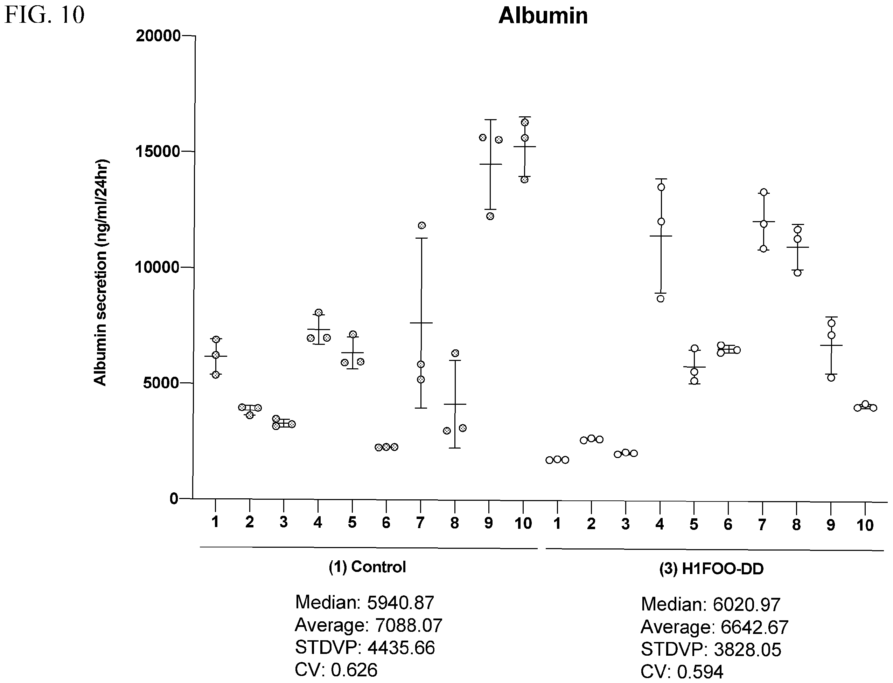

The results are shown in FIG. 10. In (3) H1FOO-DD-iPS, the secretion amount of Albumin was high on the whole as compared with (1) Control-iPS, and the median value was high. In addition, it was confirmed that the variation (STDVP, CV) between the clones is small in (3) H1FOO-DD-iPS as compared with (1) Control-iPS, and thus it is possible to more stably induce differentiation into hepatocytes. These results show that H1FOO-DD-iPS has excellent hepatocyte differentiation potency and can differentiate into hepatocytes having higher uniformity.

### [Example 12] Comparison of primitive endoderm differentiation potency

According to the method described in Example 4 described above, (1) Control-iPS and (3) H1FOO-DD-iPS, which belong to the naive type, were prepared from human skin fibroblasts and human peripheral blood mononuclear cells. Six clones (4 clones derived from human skin fibroblasts and 2 clones derived from human peripheral blood mononuclear cells) were selected for each of (1) and (3) and cultured in a primitive endoderm differentiation induction medium to induce differentiation into the primitive endoderm (PCT International Publication No. WO2019/093340). Cells were harvested on the 3rd day after the start of culture in the primitive endoderm differentiation induction medium. The expression of PDGFRA and ANPEP in the recovered cells was detected using a flow cytometer, and the proportions of PDGFRA-positive cells and ANPEP-positive cells were calculated. Here, PDGFRA and ANPEP are representative primitive endoderm markers. Each clone was subject to a test for induction of differentiation into the primitive endoderm three times.

The primitive endoderm differentiation induction medium was prepared by adding, to an N2B27 medium (NDiff227), 25 ng/mL of FGF4 (Peprotech), 1 µg/mL of Heparin (Wako), 10 ng/mL of BMP4 (R & D), 10 ng/mL of PDGFRA (Peprotech), 1 µM of XAV939 (Wako), 3 µM of A83-01 (Wako), and 0.1 µM Retinoic Acid (Sigma). On the 2nd day after the start of differentiation induction, 10 ng/mL of IL-6 (R & D) was added.

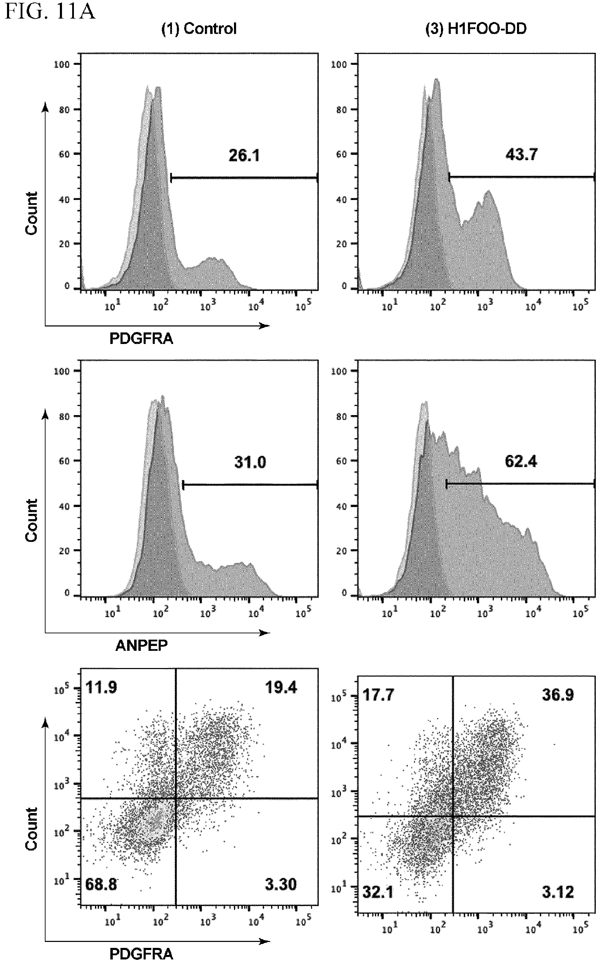

FIG. 11A shows measurement examples obtained by measuring the proportion (%) of PDGFRA-positive cells and ANPEP-positive cells by a flow cytometer. The left figure is a measurement example of (1) Control-iPS. The proportion of cells which are positive for both PDGFRA and ANPEP was about 19% of all cells.

The right figure is a measurement example of (3) H1FOO-DD-iPS. The proportion of cells which are positive for both PDGFRA and ANPEP was about 37% of all cells.

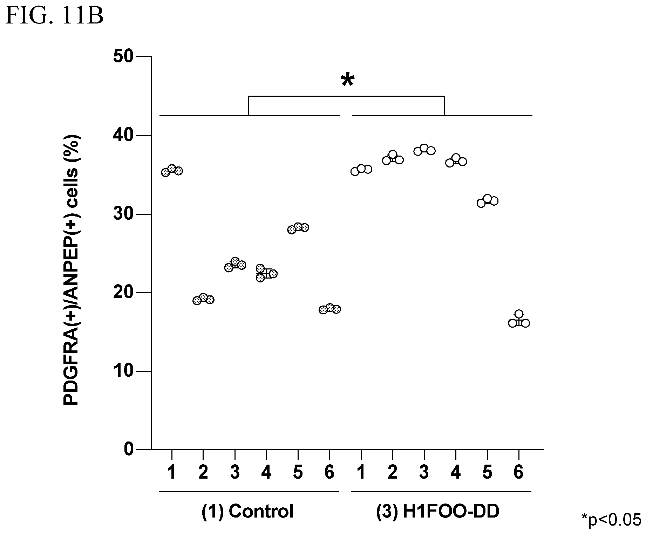

FIG. 11B shows the proportion of cells which are positive for both PDGFRA and ANPEP in each clone. In (3) H1FOO-DD-iPS, the proportion of the positive cells was high as compared with (1) Control-iPS. In addition, it was confirmed that the variation between the clones is small in (3) H1FOO-DD-iPS as compared with (1) Control-iPS, and thus it is possible to more stably induce differentiation into the primitive endoderm. These results show that H1FOO-DD-iPS has excellent primitive endoderm differentiation potency and can differentiate into the primitive endoderm having higher uniformity.

### [Example 13] Verification of metabolic function

According to the method described in Example 4 described above, (1) Control-iPS and (3) H1FOO-DD-iPS, which belong to the naive type, were prepared from human skin fibroblasts and human peripheral blood mononuclear cells. Six clones were selected for each of (1) and (3), and the spare respiratory capacity (for function evaluation of electron transport system) was measured using an extracellular flux analyzer (Agilent Seahorse XF96 Extracellular Flux Analyzer, Primetech Corporation).

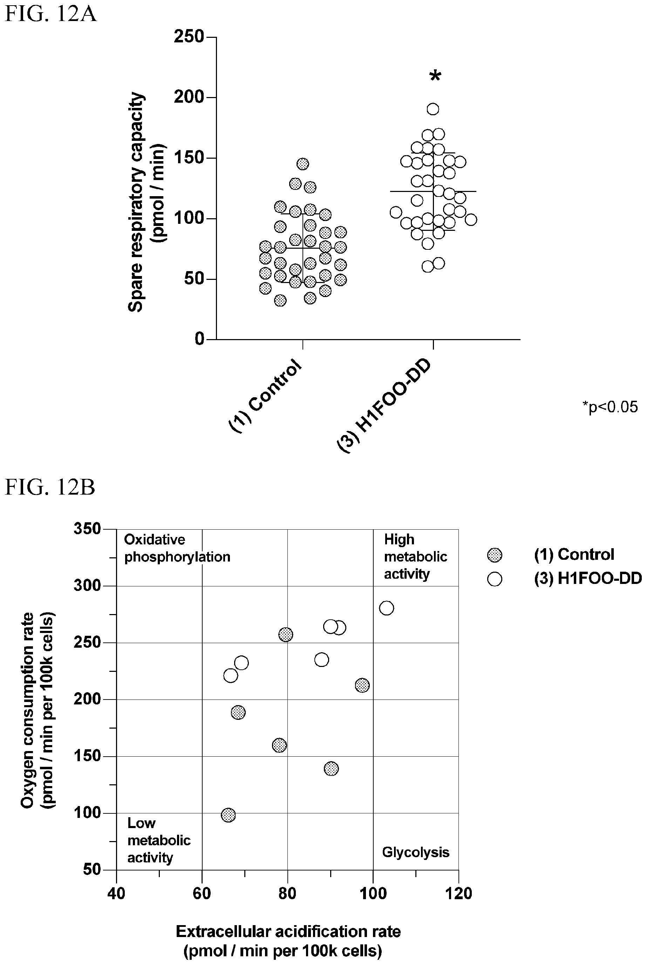

The results are shown in FIG. 12A. In (3) H1FOO-DD-iPS, the spare respiratory capacity of the positive cells was high as compared with (1) Control-iPS.

According to the method described in Example 4 above, (1) Control-iPS or (3) H1FOO-DD-iPS, which belongs to a naive type, were prepared from human skin fibroblasts or human peripheral blood mononuclear cells. Six clones were selected for each of (1) and (3), and the oxygen consumption rate (OCR, for function evaluation of electron transport system) and extracellular acidification rate (ECAR, for function evaluation of glycolysis) were measured using an extracellular flux analyzer (Agilent Seahorse XF96 Extracellular Flux Analyzer, Primetech Corporation).

The results are shown in FIG. 12B. The X-axis indicates ECAR and reflects the anaerobic metabolic capacity. The Y-axis indicates OCR and reflects the aerobic metabolic capacity. Each plot shows the average value from 6 measurements for one clone. In (3) H1FOO-DD-iPS, the plot was present in the upper right as a whole as compared with (1) Control-iPS, and both ECAR and OCR were in a high state. These results indicate that H1FOO-DD-iPS has a more naive-type metabolic capacity.

### [Example 14] Verification of presence or absence of X chromosome activation

In female-derived somatic cells and prime-type iPS/ES cells, in principle, one X chromosome is inactivated. On the other hand, it is known that both X chromosomes are activated in the naive-type preimplantation epiblast, particularly in the preimplantation epiblast. In order to verify this fact, 4 clones of each of (1) Control-iPS and (3) H1FOO-DD-iPS, which belong to the naive type, were subjected to mRNA-FISH, and the expression of three markers was compared and verified.

According to the method described in Example 4 described above, (1) Control-iPS and (3) H1FOO-DD-iPS, which belong to the naive type, were prepared from human skin fibroblasts and human peripheral blood mononuclear cells. Four clones were selected for each of (1) and (3), and the expression of UTX, HUWE1, and XIST was checked by mRNA-FISH (Sahakyan, A., et al. (2017). Cell Stem Cell 20 (1): 87-101.).

Since a large number of polyploid cells are generated in currently available naive-type human iPS cells, it is necessary to select cells having a normal number of chromosomes for correct evaluation. UTX is expressed regardless of the presence or absence of X-chromosome activation. Therefore, cells expressing two UTXs were selected as normal cells. Next, the expression HUWE1 (expressed in a case where the X chromosome is activated) and XIST (although originally involved in X chromosome inactivation, it is known to be expressed in both X chromosomes in the preimplantation epiblast) was examined by mRNA-FISH.

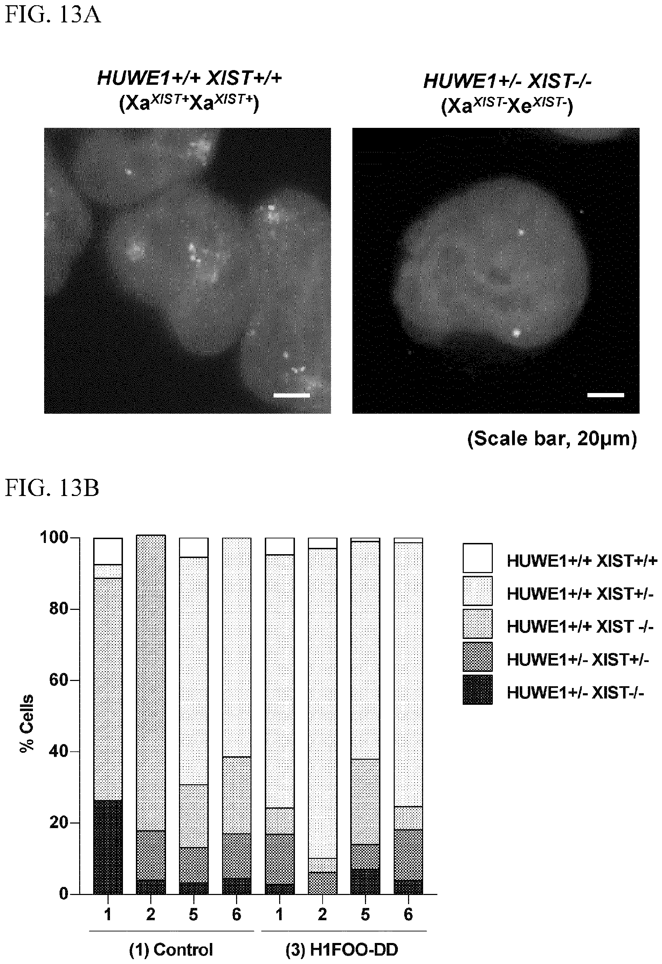

FIG. 13A shows examples of fluorescence microscopic images of mRNA-FISH. The left figure is an example of HUWEI+/+XIST+/+. The right figure is an example of HUWEI+/-XIST-/-.

FIG. 13B shows results of summarizing the expression patterns of HUWE1 and XIST for 100 cells of each clone. In FIG. 13B, the number below the bar graph indicates clone No. in (1) Control-iPS and (3) H1FOO-DD-iPS. Clone Nos. 1 and 2 are derived from skin fibroblasts, clone Nos. 5 and 6 are derived from human peripheral blood mononuclear cells.

The phenotype closest to the preimplantation epiblast is HUWE1+/+ and XIST+/+, and regarding the phenotype in accordance with this, there were clones (clones 1 and 2) dominated by a large number of cells having a phenotype close to the prime type, in (1) Control-iPS which is HUWE1+/+ and XIST+/-. On the other hand, in (3) H1FOO-DD-iPS, cells exhibiting a naive-type phenotype accounted for the majority in all the clones. These results indicate that H1FOO-DD-iPS has a phenotype closer to the naive type.

### [Example 15] Comparison of expression level of FKBP1A

Using the Sendai virus vector of (c) prepared in Example 1, H1FOO-DD (H1FOO OE HDF) was introduced into human fibroblasts (TIG120). Using a Sendai virus vector, the Oct3/4 gene, the Sox2 gene, the Klf4 gene, and the L-Myc gene were introduced into human fibroblasts (TIG120) to prepare iPS cells (OSKL). Using the Sendai virus vectors of (c) prepared in Example 1 described above, each of the above genes was introduced into human fibroblasts (TIG120) together with a Sendai virus vector containing an Oct3/4 gene, a Sox2 gene, a Klf4 gene, and an L-Myc gene, whereby H1FOO-DD-iPS (OSKLH) was prepared.

The H1FOO OE HDF, OSKL, and OSKLH prepared above were cultured, and the cells were recovered on the 2nd day from the introduction of each of the above genes. Next, the expression level of FKBP1A was measured by qRT-PCR. As controls, human fibroblasts (HDF) and H9 ES cells (H9 ESC) were subjected to the measurement of the expression level of FKBP1A. The expression level of FKBP1A was standardized by the expression level of GAPDH.

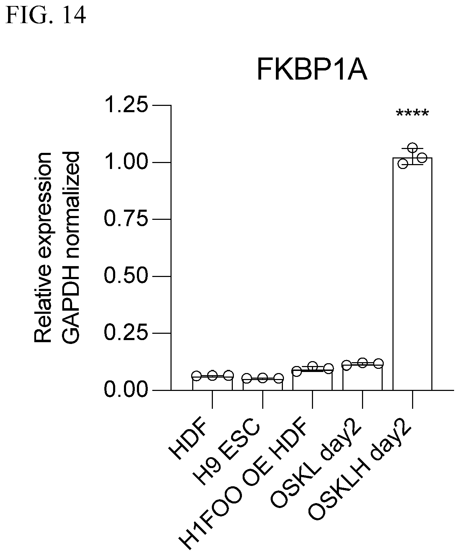

These results are shown in FIG. 14. In the figure, HDF indicates human skin fibroblasts, H9 ESC indicates H9 ES cells, H1FOO OE HDF indicates cells cultured for 2 days after the preparation of H1FOO OE HDF, OSKL day 2 indicates cells cultured for 2 days after the preparation of OSKL, and OSKLH day 2 indicates cells cultured for 2 days after the production of OSKLH. The expression level of FKBP1A was shown as a relative expression level in a case where the expression level of OSKLH day 2 was set to 1.0.

As shown in FIG. 14, the expression level of FKBP1A was significantly high only in OSKLH day 2. In OSKLH day 2, the expression level of FKBP1A was as high as about 10 times with respect to other cells. This result shows that in a case where H1FOO-DD is introduced together with a nuclear reprogramming factor, the expression level of FKBP1A increases in cells at the early stage of the nuclear reprogramming induction. Furthermore, although the results are omitted, it was confirmed that FKBP1A suppresses the expression of IFIT1 and IFNA, which are innate immune response expression markers. Therefore, it is speculated that in a case where H1FOO-DD is introduced together with a nuclear reprogramming factor, the expression of innate immune response markers is suppressed. These results suggested that the suppression of the expression of innate immune response markers may contribute to the quality improvement of iPS cells.

### [Industrial Applicability]

The present invention provides a quality improving agent for iPS cells, a method of producing iPS cells, iPS cells produced by such a production method, and a composition for producing iPS cells, which enable the production of iPS cells having good quality.

While the preferred embodiments of the present invention have been described and illustrated as described above, it is to be understood that they are intended to be illustrative and should not be considered as limiting the present invention. Additions, omissions, substitutions, and other changes can be made without departing from the spirit or scope of the present invention. Accordingly, the present invention should not be considered as being limited by the descriptions described above and is limited only by the scope of the appended claims.

## Claims

1. A quality improving agent for iPS cells, comprising:
one or more polynucleotides,
wherein the one or more polynucleotides contain an H1foo gene and a regulatory sequence that is capable of regulating in at least one of the amount and the period of existence of an H1foo protein expressed from the H1foo gene in cells when the H1foo gene is transduced into the cells.

2. The quality improving agent for iPS cells according to Claim 1,
wherein the polynucleotide is inserted into an expression vector in a state capable of expressing the H1foo gene in cells when the polynucleotide is transduced into the cells.

3. The quality improving agent for iPS cells according to Claim 2,
wherein the expression vector is a Sendai virus vector.

4. The quality improving agent for iPS cells according to any one of Claims 1 to 3,
wherein the regulatory sequence includes a nucleotide sequence encoding a destabilization domain, the destabilization domain is a domain that promotes proteasomal degradation of a fusion protein containing the destabilization domain, and
the regulatory sequence is linked to the H1foo gene such that the fusion protein of the destabilization domain and the H1foo protein is capable of being expressed.

5. The quality improving agent for iPS cells according to any one of Claims 1 to 4,
wherein the regulatory sequence includes a promoter sequence that regulates the transcription of the H1foo gene in response to a chemical stimulus.

6. A quality improving agent for iPS cells, comprising:
a fusion protein of an H1foo protein and a destabilization domain,
wherein the destabilization domain is a domain that promotes proteasomal degradation of the fusion protein.

7. A method of producing iPS cells comprising:
a step of introducing into somatic cells, a nuclear reprogramming factor and the quality improving agent for iPS cells according to any one of Claims 1 to 6.

8. The method of producing iPS cells according to Claim 7,
wherein the iPS cells are prime-type or naive-type iPS cells.

9. The method of producing iPS cells according to Claim 7 or 8,
wherein the nuclear reprogramming factor includes at least one selected from the group consisting of a gene of an Oct gene family, a gene of a Sox gene family, a gene of a Klf gene family, a gene of a Myc gene family, a gene of a Lin gene family, a Nanog gene, and a gene product thereof.

10. The method of producing iPS cells according to any one of Claims 7 to 9,
wherein the nuclear reprogramming factor consists of an Oct3/4 gene, a Sox2 gene, a Klf4 gene, L-Myc or c-Myc, and a gene product thereof.

11. The method of producing iPS cells according to any one of Claims 7 to 9,
wherein the nuclear reprogramming factor includes at least one gene selected from the group consisting of a gene of an Oct gene family, a gene of a Sox gene family, a gene of a Klf gene family, a gene of a Myc gene family, a gene of a Lin gene family, and a Nanog gene, and
the at least one gene is inserted into an expression vector in a state capable of expressing in cells when the at least one gene is transduced into the cells

12. The method of producing iPS cells according to Claim 11,
wherein the expression vector is a Sendai virus vector.

13. A method of producing iPS cells, comprising:
a step of introducing a nuclear reprogramming factor and an H1foo gene into somatic cells,
wherein an H1foo protein expressed from the H1foo gene transduced into somatic cells is regulated in at least one of the amount and the period of existence of that in the somatic cells.

14. The method of producing iPS cells according to Claim 13,
wherein the H1foo gene is inserted into an expression vector in a state capable of expressing the H1foo gene in cells when the H1foo gene is transduced into the cells.

15. The method of producing iPS cells according to Claim 13 or 14,
wherein the nuclear reprogramming factor is at least one gene selected from the group consisting of a gene of an Oct gene family, a gene of a Sox gene family, a gene of a Klf gene family, a gene of a Myc gene family, a gene of a Lin gene family, and a Nanog gene, and
the at least one gene is encoded in an expression vector that is capable of expressing the at least one gene.

16. The method of producing iPS cells according to Claim 14 or 15,
wherein the expression vector is a Sendai virus vector.

17. iPS cells that are produced by the method of producing iPS cells according to any one of Claims 7 to 16.

18. A composition for producing iPS cells, comprising:
a nuclear reprogramming factor; and
the quality improving agent for iPS cells according to any one of Claims 1 to 6.

19. A method of producing iPS cells, comprising:
a step of introducing a nuclear reprogramming factor and a substance inducing the reprogramming of somatic cells either from 2 to 15 days after being introduced into the somatic cells together with the reprogramming factor, and suppressing natural immunity, into the somatic cells.
